# EUROPÄISCHE PATENTSCHRIFT

(11) **EP 4 312 903 B1**
(45) Veröffentlichungstag und Bekanntmachung des Hinweises auf die Patenterteilung: **12.02.2025**
(21) Anmeldenummer: 22717568.4
(22) Anmeldetag: 22.03.2022
(51) Int. Cl.: A61F 5/01

(54) **FUSSORTHESE MIT DREHGELENK ZUR KORREKTUR VON FUSSFEHLSTELLUNGEN**
FOOT ORTHOSIS WITH PIVOT JOINT, FOR CORRECTING MALPOSITIONS OF THE FOOT
ORTHÈSE DE PIED À ARTICULATION ROTOÏDE POUR CORRIGER LES MALPOSITIONS DU PIED

(30) Priorität: 22.03.2021 DE 102021107084
(43) Veröffentlichungstag der Anmeldung: 07.02.2024
(73) Patentinhaber: Hallufix AG, 82031 Grünwald (DE)
(72) Erfinder: BRASS, Manfred, 82031 Grünwald (DE); OSTENRIEDER, Jörg, 82067 Ebenhausen (DE)
(74) Vertreter: Bulat, Branimir
(86) Internationale Anmeldenummer: PCT/EP2022/057525
(87) Internationale Veröffentlichungsnummer: WO 2022/200369

(56) Entgegenhaltungen:
- DE-A1- 102004 008 909
- US-A1- 2006 155 233
- US-A1- 2016 242 944
- US-A1- 2019 133 803

## Beschreibung

### Technisches Gebiet

Die vorliegende Erfindung betrifft eine Fußorthese zur Korrektur von Fußfehlstellungen, insbesondere zur Behandlung von Hallux valgus.

### Stand der Technik

Pathologische Fehlstellungen im Mittelfuß- und Vorfußbereich eines Patienten können unterschiedliche Ursachen haben, wie beispielsweise genetische Veranlagung, das Tragen von falschem Schuhwerk, insbesondere von zu engen oder hochhackigen Schuhen, oder eine Abflachung des Längs- und Quergewölbes infolge einer Instabilität des Bindegewebes im Mittelfußbereich. Insbesondere die als Hallux valgus bezeichnete Fehlstellung der Großzehe im Großzehengrundgelenk, auch als Valgusstellung bezeichnet, kommt hierbei aufgrund der stetig wachsenden Fallzahlen mehr Bedeutung zu.

Hallux valgus entsteht, indem die Großzehe im Großzehengrundgelenk durch Muskelzug in die fußinnenseitige Richtung gezogen wird. Dabei tritt der erste Mittelfußknochen als ballenförmige Ausbuchtung am Zehengrundgelenk fußinnenseitig hervor, was auch als Pseudoexostose bezeichnet wird. Die ballenförmige Ausbuchtung wird in der medizinischen Literatur auch als eine Vorwölbung im Bereich des Zehengrundgelenks oder als im Ballenbereich auftretende Schwellung beschrieben, welche bei fortschreitendem Hallux valgus zu schmerzhaften Entzündungen des hervorstehenden Zehenballens führen kann. Zudem geht mit dem Hallux valgus häufig eine Änderung der Länge und der Zugrichtung der Sehnen einher, wodurch sich die Fehlstellung im Laufe der Zeit weiter verstärken kann. Dabei entwickelt sich eine Arthrose des Großzehengrundgelenks, die im fortgeschrittenen Stadium chirurgisch behandelt werden muss.

Um den Krankheitsprozess aufzuhalten oder diesem entgegenzuwirken, sind neben chirurgischen Eingriffen auch die Anwendung konservativer Therapiemethoden bekannt. Beispielsweise ist die Verwendung von Tapeverbänden oder Orthesen bekannt zur Behandlung des Fußes in einer Ruhestellung. Aufgrund der verlangten Ruhestellung des Fußes während der Therapie werden diese vorwiegend nachts eingesetzt.

Weiterhin sind Orthesen bekannt, die in einem am Fuß befestigten Zustand einer geschienten Großzehe einen Bewegungsfreiheitsgrad entlang der Flexion-Extension-Bewegungsrichtung einräumen. Beispielsweise offenbaren die DE 102 40 121 B4 und US 2006/0155233 A1 eine orthopädische Vorrichtung in Form einer Gelenkbiegeschiene, die um eine Flexions-Extensions-Bewegungsachse der zu korrigierenden Zehe gelenkig ausgebildet ist. Hierzu ist die Gelenkbiegeschiene mit einer an der Fußinnenseite anliegenden Gelenkeinrichtung versehen, von der sich zwei Biegeschenkel entlang der Fußinnenseite erstrecken. Die Gelenkeinrichtung umfasst einen seitlich am Fuß anliegenden gewölbten Gelenkteller. Um die Gelenkbiegeschiene am Fuß zu befestigen, ist ein erster Biegeschenkel über eine erste Bandage an der Zehe und ein zweiter Biegeschenkel über eine zweite Bandage am Mittelfuß befestigt.

Weiterer Stand der Technik ist aus der US 2016/0242944 A1 und der DE 10 2004 008 909 A1 bekannt.

### Darstellung der Erfindung

Ausgehend von dem bekannten Stand der Technik ist es eine Aufgabe der vorliegenden Erfindung, eine verbesserte Fußorthese zur Korrektur von Fehlstellungen, insbesondere zur Behandlung von Hallux valgus, bereitzustellen, die insbesondere eine effektive therapeutische Behandlung sicherstellt und gleichzeitig kompakt ausgestaltet und angenehm zu tragen ist.

Die Aufgabe wird durch eine Fußorthese mit den Merkmalen des unabhängigen Anspruchs gelöst. Vorteilhafte Weiterbildungen ergeben sich aus den Unteransprüchen sowie der vorliegenden Beschreibung und den Figuren.

Entsprechend wird eine Fußorthese zur Korrektur von Fehlstellungen eines Fußes, insbesondere zur Behandlung oder Vorbeugung von Hallux valgus, bereitgestellt. Die Fußorthese umfasst eine an einer Zehe befestigbare Zehenschiene und eine im Bereich eines Mittelfußes befestigbare Mittelfußschiene, die mittels eines Drehgelenks relativ zueinander verschwenkbar gekoppelt sind. Die Fußorthese ist dazu eingerichtet, in einem an einem Fuß ordnungsgemäß befestigten Zustand über die Zehenschiene eine erste Korrekturkraft auf die Zehe auszuüben und über das Drehgelenk eine zu der ersten Korrekturkraft entgegengesetzte zweite Korrekturkraft auf ein Zehengrundgelenk auszuüben. Das Drehgelenk ist mit einer Ausnehmung in der Form einer Durchgangsöffnung entlang seiner Schwenkachse versehen und derart ausgebildet, dass in dem an dem Fuß befestigten Zustand der Fußorthese ein seitlich ausbuchtender Teil des Zehengrundgelenks in der Durchgangsöffnung zumindest teilweise aufgenommen ist. Das Drehgelenk umfasst ein mit der Zehenschiene verbundenes erstes Gelenkelement in der Form eines Gelenkrings und ein mit der Mittelfußschiene verbundenes zweites Gelenkelement in der Form eines Gelenkzapfens umfasst, die entlang der Schwenkachse und quer zu der Schwenkachse des Drehgelenks formschlüssig im Eingriff stehen, wobei der Gelenkzapfen mit einer Aufnahmenut versehen ist, in der ein dazu korrespondierender Verbindungsring des Gelenkrings geführt ist, und wobei der Gelenkzapfen an einem proximalen Ende einen umlaufenden radialen Absatz umfasst, der in Richtung der Schwenkachse eine formschlüssige Verbindung zwischen dem Gelenkring und dem Gelenkzapfen bereitstellt und in einer korrespondierend ausgebildeten weiteren Ausnehmung am Gelenkring aufgenommen ist.

Indem die vorgeschlagene Fußorthese neben der auf die Zehe wirkenden ersten Korrekturkraft zusätzlich die auf das Zehengrundgelenk mittels des Drehgelenks wirkende zweite Korrekturkraft bereitstellt, kann eine besonders effektive therapeutische Wirkung erzielt werden. Denn durch die vorgeschlagene Ausgestaltung der Fußorthese kann gleichzeitig eine therapeutische Wirkung auf eine Valgusstellung der Zehe als auch auf eine Varusstellung des Zehengrundgelenks erzielt werden. Somit können Symptomatik und Ursache der Fußfehlstellung gleichzeitig behandelt werden. Die auf den Fuß wirkenden Korrekturkräfte und damit einhergehenden therapeutischen Effekte werden nachstehend im Zusammenhang mit den damit in Verbindung stehenden Komponenten der Fußorthese genauer beschrieben.

Im Rahmen der vorliegenden Erfindung wurde erkannt, dass der seitlich ausbuchtende Teil des Zehengrundgelenks, insbesondere beim Vorhandensein der Pseudoexostose, wie diese bei Hallux valgus vorkommt, sensibel und druckempfindlich ist. Um diesen Umstand Rechnung zu tragen, ist die vorgeschlagene Fußorthese mit dem mit der Ausnehmung versehenen Drehgelenk ausgestattet, in dessen Ausnehmung der seitlich ausbuchtende Teil des Zehengrundgelenks beim Tragen der Fußorthese zumindest teilweise aufgenommen ist. Dadurch kann verhindert werden, dass die Fußorthese an einem distalen Ende des seitlich ausbuchtenden Teils des Zehengrundgelenks anliegt und auf diesen Kräfte unmittelbar ausübt. So kann im Vergleich zu bekannten Orthesen, die an dem seitlich ausbuchtenden Teil des Zehengrundgelenks anliegen und auf diesen unmittelbar Kräfte ausüben, die auf den sensiblen und druckempfindlichen Teil des Zehengrundgelenks wirkende Belastung beim Tragen verringert werden. Mit anderen Worten ermöglicht die vorgeschlagene Fußorthese durch die geometrische Ausgestaltung des Drehgelenks eine Reduzierung oder Vermeidung von Belastungen auf druckempfindliche Bereiche des Fußes.

Weiterhin ermöglicht die Ausgestaltung des vorgeschlagenen Drehgelenks, dass die Fußorthese enger am Fuß geführt werden kann und deren Erstreckung in Fußbreitenrichtung verringert werden kann. Dies gilt insbesondere für jene Bereiche oder Abschnitte der Fußorthese, welche im am Fuß ordnungsgemäß befestigten Zustand seitlich an dem Zehengrundgelenk des Trägers angeordnet sind. Entsprechend kann das vorgeschlagene Drehgelenk zu einer kompakten Bauform der Fußorthese beitragen. Das Tragen der vorgeschlagenen Fußorthese in üblichem Schuhwerk kann so für einen Patienten gegenüber bekannten Vorrichtungen deutlich angenehmer sein und durch die kompakte Bauweise überhaupt erst ermöglicht werden.

Die vorgeschlagene Fußorthese ist dafür vorgesehen, eine pathologische Fehlstellung eines Fußes, insbesondere einer Zehe und/oder eines Zehengrundgelenks, zu behandeln, entgegenzuwirken und/oder vorzubeugen. Insbesondere kann die Fußorthese zur Vorbeugung oder Behandlung von Hallux valgus eingesetzt werden, ist aber nicht auf diese Anwendung beschränkt. Entsprechend ist die Fußorthese dafür vorgesehen, an einem Fuß eines Patienten befestigt zu werden und in dem am Fuß befestigten Zustand auf den Fuß, insbesondere auf die Zehe und/oder das Zehengrundgelenk, therapeutisch einzuwirken.

In der vorliegenden Offenbarung bezieht sich der Ausdruck "in einem/dem am Fuß ordnungsgemäß befestigten Zustand", vorliegend auch mit "in dem befestigten Zustand" abgekürzt, auf einen Zustand, in dem die Fußorthese an einem Fuß eines Patienten bestimmungsgemäß befestigt ist und entsprechend einen gewünschten therapeutischen Effekt zur Korrektur oder zur Vorbeugung von Fehlstellungen bewirkt. Die Fußorthese kann fußspezifisch für einen linken oder rechten Fuß eines Patienten ausgestaltet sein. Mit anderen Worten kann die Fußorthese entweder zur Verwendung an dem linken oder dem rechten Fuß des Patienten vorgesehen und ausgebildet sein. Eine für den linken Fuß vorgesehene Fußorthese kann spiegelsymmetrisch zu einer für den rechten Fuß eines Patienten vorgesehenen Fußorthese ausgebildet sein.

Die vorgeschlagene Fußorthese ist derart ausgestaltet, dass sie in dem befestigten Zustand Korrekturkräfte auf den Fuß ausübt. Unter dem Begriff "Korrekturkräfte" werden vorliegend solche Kräfte verstanden, die eine therapeutische Wirkung auf den zu behandelnden Fuß haben. Insbesondere bewirken die Korrekturkräfte, dass die von der Fehlstellung betroffenen Teile des Fußes in eine anatomisch korrekte oder beabsichtigte Position gelenkt werden, um einen gewünschten therapeutischen Effekt zu erzielen.

Die vorgeschlagene Fußorthese ist dazu eingerichtet, in dem am Fuß des Patienten befestigten Zustand wenigstens die erste und zweite Korrekturkraft mittels der Zehenschiene und der Mittelfußschiene auf den zu behandelnden Fuß auszuüben, insbesondere unmittelbar auszuüben. Durch diese Ausgestaltung unterscheidet sich die vorliegende Fußorthese wesentlich von bekannten Vorrichtungen, die ein Zehengrundgelenk und den diesem zugeordneten Zehenballen oder eine durch die Fehlstellung bewirkte pathologische Pseudoexostose vor äußerer Krafteinwirkung, insbesondere von durch die Vorrichtung auf den Fuß wirkenden Kräften, gewollt oder ungewollt abschirmen. Wir vorrangehend beschrieben wurde im Rahmen der vorliegenden Fußorthese erkannt, dass eine besonders effektive therapeutische Wirkung erzielt werden kann, indem die Fußorthese neben der auf die Zehe wirkenden ersten Korrekturkraft zusätzlich die auf das Zehengrundgelenk wirkende zweite Korrekturkraft ausübt. Das dadurch resultierende Zusammenspiel von auf den Fuß ausgeübten Korrekturkräften kann besonders vorteilhaft bei der Behandlung von Hallux valgus sein.

Im Zusammenhang mit der Zehenschiene wird nachfolgend zur Vereinfachung allgemein auf eine Zehe des Fußes Bezug genommen. Hierbei handelt es sich insbesondere um die Großzehe des zu behandelnden Fußes. Die Fußorthese ist hierauf aber nicht beschränkt, sodass mit dem Ausdruck beispielsweise auch die Kleinzehe gemeint sein kann. Im Zusammenhang mit dem Drehgelenk und der damit in Verbindung stehenden zweiten Korrekturkraft wird entsprechend zur Vereinfachung allgemein auf ein Zehengrundgelenk Bezug genommen, wobei insbesondere das Großzehengrundgelenk gemeint ist, die Fußorthese hierauf aber nicht beschränkt ist. Alternativ kann es sich beispielsweise auch um das Kleinzehengrundgelenk handeln.

In der vorliegenden Offenbarung wird zur Beschreibung der Fußorthese, insbesondere in Bezug auf den zu behandelnden Fuß, ein Bezugssystem verwendet, das auf die Mittellinie des Körpers eines Patienten ausgerichtet ist, wie in der Anatomie üblich. So können die Position und Richtung der einzelnen Komponenten der vorgeschlagenen Fußorthese in dem befestigten Zustand in Bezug auf den in der Fußorthese aufgenommenen Fuß angegeben werden. Dementsprechend bezieht sich der Begriff "medial" auf eine Richtung oder Seite der Fußorthese, die in Richtung einer medialen Ebene des Körpers des Trägers zeigt. In der Anatomie bezieht sich der Begriff "mediale Ebene", die auch als "Mittelsagittalebene" bezeichnet wird, im Allgemeinen auf eine anatomische Ebene, die den Körper in zwei symmetrische Teile teilt. Dementsprechend bedeutet der Begriff "in medialer Richtung" bei der Beschreibung einer Fußorthese eine Richtung, die von dem zu behandelnden Fuß des Patienten in Richtung seines anderen Fußes zeigt. In diesem Sinne bezieht sich der Begriff "lateral" auf eine Richtung oder Seite der Fußorthese, die von der medialen Ebene des Körpers des Trägers weg weist. Dementsprechend bedeutet der Begriff "in lateraler Richtung" bei der Beschreibung einer Fußorthese, welche an einem Fuß des Trägers befestigt ist, in einer Richtung, die von dem anderen Fuß des Trägers weg weist.

Zur Interaktion mit dem Zehengrundgelenk des zu behandelnden Fußes, das heißt zum Ausüben der zweiten Korrekturkraft, ist die Fußorthese mit dem Drehgelenk ausgestattet, das insbesondere als Hohlzapfendrehgelenk ausgeführt ist. Vorliegend wird als "Drehgelenk" ein Gelenk verstanden, bei dem zwei relativ zueinander verschwenkbar und in Eingriff gebrachte Komponenten des Gelenks relativ zueinander verschwenkbar oder rotierbar gelagert sind. Im Zusammenhang mit der vorliegenden Offenbarung betrifft der Begriff "Hohlzapfendrehgelenk", das auch als nabenloses Drehgelenk bezeichnet werden kann, ein Gelenk, das entlang seiner Schwenkachse zumindest abschnittsweise hohl ausgebildet ist, um die Ausnehmung auszubilden. Mit anderen Worten sind die das Drehgelenk bildenden Komponenten entlang der Schwenkachse hohl ausgebildet, sodass das Drehgelenk um und entlang dessen Schwenkachse mit der Ausnehmung oder Durchgangsöffnung versehen ist.

Das Drehgelenk kann relativ zueinander um die Schwenkachse verschwenkbare Komponenten aufweisen, die relativ zueinander in dem Drehgelenk geführt sind. Der für die Führung der Komponenten vorgesehene Bereich, der durch Lagerpunkte und/oder Lagerflächen, insbesondere durch Kontaktflächen und/oder Gleitflächen, gebildet sein kann, ist vorzugsweise umlaufend um die Schwenkachse und beabstandet zu dieser angeordnet.

Der für die Führung der Komponenten des Drehgelenks vorgesehene Bereich kann um einen Führungsradius beabstandet zu der Schwenkachse angeordnet sein. Der Führungsradius kann insbesondere einen mittleren Radius des für die Führung der Komponenten des Drehgelenks vorgesehenen Bereichs um die Schwenkachse angeben. Der Führungsradius kann im Wesentlichen im Bereich eines Außenradius des Drehgelenks liegen, der eine Erstreckung des Drehgelenks in radialer Richtung beschreibt. Der Führungsradius kann wenigstens 70% des Außenradius des Drehgelenks betragen. Im Speziellen kann der Führungsradius wenigstens 80% oder wenigstens 90% des Außenradius betragen.

Das Drehgelenk kann in Form eines Ringgelenks bereitgestellt sein, bei dem die Führungsfläche zwischen den relativ zueinander verschwenkbaren Komponenten des Drehgelenks ringförmig um die Gelenk- oder Schwenkachse des Drehgelenks angeordnet sind/ist. Auf diese Weise kann das Drehgelenk besonders robust gegenüber Biegekräften und Biegemomenten sein, sodass das Drehgelenk hohe Kräfte und Momente übertragen und in den zu behandelnden Fuß einleiten kann, bei einer gleichzeitig kompakten Bauweise der Fußorthese.

Das Drehgelenk kann derart ausgebildet sein, dass in dem befestigten Zustand der Fußorthese das Drehgelenk derart an dem Fuß und dem Zehengrundgelenk angeordnet ist, dass das Drehgelenk umlaufend um den seitlich ausbuchtenden Teil des Zehengrundgelenks, beispielsweise um die Pseudoexostose, angeordnet sein kann, wobei der seitlich ausbuchtende Teil des Zehengrundgelenks zumindest teilweise in der Ausnehmung des Drehgelenks angeordnet ist.

Die Ausnehmung ist in Form einer Durchgangsöffnung, insbesondere entlang der Schwenkachse ausgebildet. Mit anderen Worten kann sich die Ausnehmung entlang der gesamten Breite oder Dicke des Drehgelenks erstrecken. Vorliegend betreffen die Begriffe "Breite" oder "Dicke" des Drehgelenks eine Erstreckung des Drehgelenks entlang der Schwenkachse. Das Drehgelenk kann eine Breite, insbesondere maximale Breite, von höchstens 1,0 cm oder von höchstens 0,6 cm aufweisen.

Die Fußorthese kann derart ausgestaltet sein, dass in dem befestigten Zustand der seitlich ausbuchtende Teil des Zehengrundgelenks sich entlang wenigstens 50% oder wenigstens 70% oder wenigstens 80% der maximalen Breite des Drehgelenks erstreckt. Weiterhin kann die Fußorthese derart ausgestaltet sein, dass in dem befestigten Zustand der seitlich ausbuchtende Teil des Zehengrundgelenks durch die Ausnehmung, insbesondere die Durchgangsöffnung, entlang der Schwenkachse hindurchragt oder im Wesentlichen hindurchragt. Auf diese Weise kann die Fußorthese besonders eng am Fuß geführt werden.

Das Drehgelenk kann eine die Ausnehmung begrenzende und radial um die Schwenkachse angeordnete Seitenwand umfassen. Die Seitenwand kann einen minimalen Krümmungsradius von 1 mm oder 2 mm oder 5 mm aufweisen. Mit anderen Worten kann das Drehgelenk derart ausgebildet sein, dass die Seitenwand an keiner Stelle einen Krümmungsradius aufweist, der unterhalb des minimalen Krümmungsradius liegt. Der Kehrwert des Krümmungsradius entspricht hierbei der Krümmung der Seitenwand, insbesondere deren der Ausnehmung zugewandten Innenfläche. Auf diese Weise kann sichergestellt werden, dass der an dem seitlich ausbuchtenden Teil des Zehengrundgelenks anliegende Bereich des Drehgelenks nicht mit spitzen Kanten versehen ist, um so Druckspitzen am Fuß des Patienten beim Tragen der Fußorthese zu verhindern.

Die Ausnehmung kann einen minimalen Durchmesser, insbesondere entlang einer Richtung quer zur Schwenkachse oder um die Schwenkachse, von wenigstens 1,5 cm oder wenigstens 2,0 cm oder wenigstens 2,5 cm betragen. Beispielsweise kann die Ausnehmung im Querschnitt entlang der Schwenkachse eine kreisförmige oder elliptische Form aufweisen, deren minimaler Durchmesser wenigstens 1,5 cm oder wenigstens 2,0 cm oder wenigstens 2,5 cm betragen kann. Beispielsweise kann der Durchmesser 3,0 cm oder im Wesentlichen 3,0 cm betragen.

Die Form der Ausnehmung, insbesondere deren Querschnittsform und Durchmesser, kann an den zu behandelnden Fuß, insbesondere an die Form des seitlich ausbuchtenden Teils des Zehengrundgelenks, angepasst sein. Dies kann aufgrund orthopädischer beziehungsweise physiologischer Klassifikation anwendergruppenspezifisch erfolgen. Beispielsweise können auf diese Weise Fußorthesen für Anwendergruppen mit unterschiedlich großen Füßen und/oder unterschiedlich großen seitlich ausbuchtenden Teilen des Zehengrundgelenks beziehungsweise Pseudoexostosen bereitgestellt werden.

Wie vorangehend beschrieben, sind die Zehenschiene und die Mittelfußschiene mittels des Drehgelenks relativ zueinander verschwenkbar gekoppelt. Auf diese Weise wird ermöglicht, dass beim Tragen der Fußorthese die mittels der vorgeschlagenen Fußorthese geschiente Zehe entlang der Flexion-Extension-Bewegungsrichtung bewegt werden kann relativ zu dem Mittelfuß. Mit anderen Worten kann die Fußorthese und entsprechend das Drehgelenk derart eingerichtet sein, dass in dem am Fuß befestigten Zustand der Fußorthese die zu behandelnde Zehe relativ zu dem Großzehengrundgelenk in Flexion-und Extensionsrichtung bewegbar ist. Entsprechend kann das Drehgelenk derart eingerichtet sein, dass in dem am Fuß befestigten Zustand die Schwenkachse des Drehgelenks parallel oder im Wesentlichen parallel zu einer Grundgelenkachse des Zehengrundgelenks ist, insbesondere parallel oder im Wesentlichen parallel zu der Flexions-Extensions-Bewegungsachse des Zehengrundgelenks ist. Weiterhin kann die Schwenkachse des Drehgelenks mit der Grundgelenkachse des Zehengrundgelenks, insbesondere mit der Flexions-Extensions-Bewegungsachse, fluchten, d.h. mit dieser zusammenfallen, oder im Wesentlichen fluchten. Alternativ oder zusätzlich kann die Schwenkachse des Drehgelenks parallel oder im Wesentlichen parallel zu der ersten und/oder zweiten Korrekturkraft angeordnet sein.

Weiterhin kann das Drehgelenk dazu eingerichtet sein, Scher- und/oder Biegekräfte zwischen der Zehenschiene und der Mittelfußschiene zu übertragen, um die erste und/oder zweite Korrekturkraft auf den Fuß auszuüben. Mit anderen Worten kann das Drehgelenk der Fußorthese dazu eingerichtet sein, in dem befestigten Zustand parallel zu den Korrekturkräften wirkende Kräfte, insbesondere Scherkräfte oder Schubkräfte, zu übertragen, um die erste und/oder zweite Korrekturkraft zu generieren. Hierzu kann das Drehgelenk dazu ausgebildet sein, zwischen der Mittelfußschiene und der Zehenschiene Kräfte in Richtung der Schwenkachse des Drehgelenks zu übertragen. Insbesondere kann das Drehgelenk derart ausgebildet sein, zwischen der Mittelfußschiene und der Zehenschiene entlang einer Längserstreckung der Fußorthese, welche von der Mittelfußschiene über das Drehgelenk hin zur Zehenschiene verläuft und welche entsprechend im an dem Fuß angebrachten Zustand der Fußorthese im Wesentlichen in Richtung der Längsrichtung des Fußes entspricht, Biegekräfte zu übertragen. Die Biegekräfte können in medial-lateral Richtung und/oder lateral-medial Richtung verlaufen. Die so entlang der Schiene übertragenen Kräfte können die durch die Fußorthese auf den Fuß auszuübenden Korrekturkräfte bewirken.

Auf diese Weise räumt die vorgeschlagene Fußorthese einer geschienten Zehe eine ausreichende Bewegungsfreiheit ein, sodass die Fußorthese den Fuß in seiner natürlichen Gehbewegung unterstützt und gleichzeitig therapeutisch auf diesen einwirkt. Dies ermöglicht den Einsatz der vorgeschlagenen Fußorthese im Alltag des Patienten, was dessen Bereitschaft zum Tragen der Fußorthese und daher die Akzeptanz und damit einhergehend den Erfolg der therapeutischen Behandlung erhöht.

Das Drehgelenk kann derart ausgebildet sein, dass in dem am Fuß befestigten Zustand der Fußorthese eine relative Schwenkbewegung zwischen der Zehenschiene und der Mittelfußschiene um eine zu der Schwenkachse schräg oder orthogonal angeordnete Achse gesperrt ist. Das Drehgelenk kann derart ausgebildet sein, dass eine relative Schwenkbewegung zwischen der Zehenschiene und der Mittelfußschiene nur um die Schwenkachse zugelassen ist. Mit anderen Worten kann das Drehgelenk strukturell derart ausgebildet sein, dass Schwenkbewegungen um die Schwenkachse erfolgen können, aber Schwenkbewegungen um eine Achse schräg oder quer zu der Schwenkachse gesperrt sind. Auf diese Weise kann eine einfache und kompakte Bauweise des Drehgelenks bereitgestellt sein.

Die Fußorthese ist vorzugsweise derart ausgebildet, dass in dem am Fuß befestigten Zustand das Drehgelenk seitlich am Fuß angeordnet ist. Insbesondere kann das Drehgelenk an einer medialen Seite des Fußes in dem befestigten Zustand angeordnet sein. Mit anderen Worten kann das Drehgelenk in dem am Fuß befestigten Zustand fußinnenseitig, also medial am Fuß angeordnet sein, wobei insbesondere die Zehenschiene und die Mittelfußschiene medial angeordnet sein können.

Das Drehgelenk kann durch miteinander strukturell im Eingriff stehende Bereiche der Zehenschiene und der Mittelfußschiene gebildet sein. Insbesondere kann das Drehgelenk durch miteinander strukturell im Eingriff stehende Endabschnitte der Zehenschiene und der Mittelfußschiene gebildet sein. Das Drehgelenk kann wenigstens teilweise durch die im Eingriff stehenden Bereiche der Zehenschiene und der Mittelfußschiene gebildet sein oder ausschließlich durch die im Eingriff stehende Bereiche der Zehenschien und der Mittelfußschiene gebildet sein. Der das Drehgelenk bildende Teil der Zehenschiene und/oder der Mittelfußschiene kann integraler Bestandteil der Zehenschiene und/oder der Mittelfußschiene sein. Auf diese Weise kann ein einfacher Aufbau der Fußorthese durch die Verwendung einer geringen Anzahl von Komponenten sichergestellt werden.

Gemäß einer Weiterbildung der Fußorthese kann das Drehgelenk ein mit der Zehenschiene gekoppeltes, insbesondere integral oder stoffschlüssig verbundenes, erstes Gelenkelement und ein dazu komplementär ausgebildetes und im Eingriff, bevorzugt im direktem Eingriff, stehendes zweites Gelenkelement umfassen, das mit der Mittelfußschiene gekoppelt ist, insbesondere integral oder stoffschlüssig verbunden ist. Das erste Gelenkelement und das zweite Gelenkelement können entlang der Schwenkachse, insbesondere in einer ersten und einer dazu entgegengesetzten zweiten Richtung entlang der Schwenkachse, und/oder quer zu der Schwenkachse des Drehgelenks formschlüssig im Eingriff stehen.

Das Drehgelenk kann derart ausgestaltet sein, dass in dem befestigten Zustand das zweite Gelenkelement zwischen dem Fuß und dem ersten Gelenkelement angeordnet ist. Auf diese Weise kann verhindert werden, dass bei einer Beugebewegung der geschienten Zehe, der an dem Fuß anliegende Teil des Drehgelenks relativ zu dem seitlich ausbuchtenden Teil verschwenkt wird. Dies kann den Tragekomfort der Fußorthese erhöhen.

Das zweite Gelenkelement kann den Gelenkzapfen des Drehgelenks bilden, der das den Gelenkring bildende erste Gelenkelement um die Schwenkachse führt. Der Gelenkzapfen ist vorzugsweise als Hohlzapfen ausgebildet, wobei der hohle Teil des Hohlzapfens die Ausnehmung bildet. Der Gelenkring und der Gelenkzapfen sind derart ausgebildet und stehen derart im Eingriff, dass diese entlang der Schwenkachse, insbesondere in der ersten und der dazu entgegengesetzten zweiten Richtung entlang der Schwenkachse, formschlüssig im Eingriff stehen.

Der Gelenkzapfen kann in seiner geometrischen Ausgestaltung an die Form des Gelenkrings angepasst sein. Der Gelenkring kann eine erste Führungsfläche aufweisen, die korrespondierend zu einer zweiten Führungsfläche des Gelenkzapfens ausgestaltet sein kann. Die erste und zweite Führungsfläche, die insbesondere Gleit- und Lagerflächen bilden, können im Eingriff stehen, insbesondere im Wesentlichen spielfrei oder mit einem vorgegebenen Spiel. Bei einer Schwenkbewegung können die erste und zweite Führungsfläche relativ zueinander bewegt werden.

Die erste Führungsfläche des Gelenkrings kann eine radial nach innen gerichtete, d.h. der Schwenkachse zugewandte, Fläche sein oder umfassen. Die erste Führungsfläche kann sich entsprechend in Umfangsrichtung um die Schwenkachse erstrecken und ringförmig um die Schwenkachse angeordnet sein.

Die zweite Führungsfläche des Gelenkzapfens kann eine radial nach außen gerichtete Fläche sein oder umfassen und insbesondere eine Mantelfläche des Gelenkzapfens bilden. Die zweite Führungsfläche kann sich entsprechend in Umfangsrichtung um die Schwenkachse erstrecken und ringförmig um die Schwenkachse angeordnet sein.

Der Gelenkzapfen umfasst an einem proximalen Ende einen umlaufenden radialen Absatz, welcher vorzugsweise, insbesondere im Sinne eines Schnapphakens, in Richtung der Drehachse eine formschlüssige Verbindung bzw. hinterschnittige Sicherung zwischen dem Gelenkring und Gelenkzapfen bereitstellt. Um an der medialen Außenseite der Fußorthese eine im Wesentlichen ebene Außenkontur bzw. Außenfläche bereitzustellen, kann der radiale Absatz in der korrespondierend ausgebildeten weiteren Ausnehmung am Gelenkring aufgenommen sein. Alternativ oder zusätzlich kann ein separater Sicherungsring vorgesehen sein, welcher in eine entsprechende Nut am Gelenkring oder Gelenkzapfen eingesetzt werden kann. Der radiale Absatz kann sich entlang der Schwenkachse derart erstrecken, dass dieser in Axialrichtung des Drehgelenks überlappend zu dem Gelenkring angeordnet ist und diesen insbesondere umgreift. Durch diese Ausgestaltung kann verhindert werden, dass der zu behandelnde Fuß mit der Aufnahmenut in Kontakt tritt, um den Tragekomfort für einen Patienten zu erhöhen.

Die erste und zweite Führungsfläche können wenigstens eine, beispielsweise zwei gegenüberliegenden axial begrenzende Seitenflächen umfassen, um die formschlüssige Verbindung entlang der Schwenkachse bereitzustellen. Auf diese Weise können Kräfte, insbesondere Biegekräfte in Richtung der Schwenkachse des Drehgelenks zwischen den Komponenten übertragen werden.

Gemäß einer Ausgestaltung kann der Gelenkzapfen, insbesondere das zweite Gelenkelement, oder der Gelenkring mit einer um die Schwenkachse umlaufend angeordneten Aufnahmenut versehen sein, die sich in radialer Richtung erstreckt, d.h. zu der Schwenkachse hin erstreckt, und in axialer Richtung entlang der Schwenkachse begrenzt ist, d.h. seitlich begrenzt ist. Die Innenflächen der Aufnahmenut bilden hierbei eine Kontakt- und Gleitfläche, d.h. die erste oder zweite Führungsfläche. Mit anderen Worten kann die Aufnahmenut im Längsschnitt entlang der Schwenkachse eine im Wesentlichen U-förmige Kontakt- oder Gleitfläche ausbilden. Die dazu korrespondierende Führungsfläche kann ein zu der Aufnahmenut korrespondierend ausgebildeter Verbindungsring sein, der in der Aufnahmenut geführt ist und relativ zu der Aufnahmenut in Umfangsrichtung um die Schwenkachse verdrehbar ist. In dem im Eingriff stehenden Zustand der Aufnahmenut und des Verbindungsrings ist der Verbindungsring derart in der Aufnahmenut angeordnet, dass das erste Gelenkelement und das zweite Gelenkelement formschlüssig in Axialrichtung der Schwenkachse miteinander verbunden sind.

Mit anderen Worten kann der Gelenkzapfen mit einer Aufnahmenut versehen sein, in der ein dazu korrespondierender Verbindungsring des Gelenkrings geführt ist. Alternativ kann der Gelenkring mit der Aufnahmenut versehen sein, in der der dazu korrespondierende Verbindungsring des Gelenkzapfens geführt ist.

Die Fußorthese umfasst ferner die Zehenschiene. Die Zehenschiene kann dafür vorgesehen sein, an der Zehe des Fußes an einer vorgegebenen Position in Eingriff gebracht zu werden, um in dem am Fuß befestigten Zustand der Fußorthese eine kraftübertragende Kopplung zwischen der Zehe und der Zehenschiene bereitzustellen. Entsprechend wird die Zehenschiene in dem am Fuß befestigten Zustand der Fußorthese in einer gewünschten Position an dem Fuß gehalten. Die Zehenschiene ist ferner dafür vorgesehen, in dem am Fuß befestigten Zustand die erste Korrekturkraft auf die Zehe auszuüben. Die erste Korrekturkraft kann auf die Zehe in medialer Richtung wirken, wobei die durch das Drehgelenk ausgeübte Korrekturkraft in lateraler Richtung wirken kann.

Die Fußorthese umfasst ferner die Mittelfußschiene. Die Mittelfußschiene kann dafür vorgesehen sein, in dem am Fuß befestigten Zustand eine Haltekraft auf den Mittelfuß auszuüben. Die Haltekraft kann in einer Richtung parallel zu der ersten Korrekturkraft wirken und zusammen mit der ersten Korrekturkraft eine Gegenkraft zu der zweiten Korrekturkraft bilden. Durch das Zusammenwirken dieser Kräfte kann die Fußorthese zuverlässig an dem zu behandelnden Fuß in einer für die therapeutische Behandlung vorgesehenen Position stabil gehalten werden. Durch diese Ausgestaltung kann die Fußorthese mit der Wirkungsweise einer Spannklemme oder -klammer an dem zu behandelnden Fuß befestigt werden.

In einer Weiterentwicklung kann die durch die Mittelfußschiene auszuübende Haltekraft eine therapeutische Wirkung auf den zu behandelnden Fuß haben, die insbesondere zur therapeutischen Wirkung der ersten und zweiten Korrekturkraft beiträgt und/oder eine davon zu unterscheidende, weitere therapeutische Wirkung bereitstellt. Beispielsweise kann die durch die Mittelfußschiene ausgeübte weitere Korrekturkraft ein Aufrichten des Fußgewölbes bewirken. Zur Unterstützung dieser Wirkung, kann die Fußorthese weiterhin ein unterhalb der Fußsohle, insbesondere des Fußgewölbes im Mittelfußbereich anzuordnendes Fußkissen, auch als Pelotte bezeichnet, umfassen. Ein derartiges Fußkissen kann lösbar mit der Mittelfußschiene verbunden sein.

Die Zehenschiene und/oder die Mittelfußschiene kann/können ein Bügelelement umfassen, das sich entlang der zu schienenden Zehe oder entlang des zu schienenden Mittelfußes erstreckt.

Unter dem Begriff "Bügelelement" wird vorliegend und im Allgemeinen eine Komponente verstanden, die dazu eingerichtet und dafür vorgesehen ist, unterschiedliche Belastungen aufzunehmen und zu übertragen, wie zum Beispiel Längskräfte, Querkräfte, Scherkräfte, Biegekräfte, Biegemomente, Torsionsmomente, etc. So ist ein Bügelelement dafür vorgesehen, neben Zugkräften auch Druckkräfte entlang seiner Längsachse und Querkräfte quer zu seiner Erstreckungsachse, insbesondere seiner Längsachse, aufzunehmen und zu übertragen. Diese Eigenschaft unterscheidet einen Bügel wesentlich von einer Bandage, die zwar zur Übertragung von Zugkräften, aber nicht zur Übertragung von Druck- und/oder Querkräften vorgesehen ist.

Entsprechend kann das Bügelelement der Zehenschiene und/oder der Mittelfußschiene derart ausgebildet sein, in dem am Fuß befestigten Zustand Scherkräfte und/oder Biegekräfte, insbesondere in Richtung der ersten und zweiten Korrekturkraft, aufzunehmen und zu übertragen um die erste Korrekturkraft auf die Zehe auszuüben und/oder die Haltekraft auf den Mittelfuß auszuüben.

Gemäß einer Weiterentwicklung kann das Bügelelement der Zehenschiene und/oder der Mittelfußschiene in Form eines Spannbügels oder einer Biegefeder ausgebildet sein. In dieser Ausgestaltung kann die erste Korrekturkraft und/oder die zweite Korrekturkraft und/oder die Haltekraft in Form einer durch elastische Verformung des Bügelelements der Zehenschiene und/oder der Mittelfußschiene induzierten Spannkraft bereitgestellt sein.

Entsprechend kann die Fußorthese derart eingerichtet sein, dass in dem an dem Fuß befestigten Zustand das Drehgelenk zu der ersten oder zweiten Korrekturkraft parallele Biegekräfte zwischen der Mittelfußschiene und der Zehenschiene überträgt, wobei die erste Korrekturkraft und/oder die zweite Korrekturkraft und/oder die Haltekraft in Form einer durch eine elastische Verformung der Zehenschiene und der Mittelfußschiene induzierte Biegekraft bereitgestellt ist. Auf diese Weise kann sichergestellt werden, dass auf die Zehe und das Zehengrundgelenk beim Tragen der Fußorthese anhaltend die Korrekturkräfte ausgeübt werden, d.h. auch dann, wenn sich die Zehe relativ zum Mittelfuß bewegt, wie dies beispielsweise beim Gehen der Fall ist.

Die Fußorthese kann derart ausgestaltet sein, dass in einem vom Fuß entkoppelten Zustand der Fußorthese, d.h. in einem Zustand, in dem die Fußorthese in keinem Eingriff mit dem zu behandelnden Fuß steht und entsprechend freiliegt, die Fußorthese, insbesondere das Bügelelement der Zehenschiene und/oder die Mittelfußschiene, in einer Ruheposition angeordnet ist, in der die Fußorthese, insbesondere das Bügelelement der Zehenschiene und/oder der Mittelfußschiene, nicht elastisch verformt ist. In dem am Fuß befestigten Zustand der Fußorthese kann die Fußorthese, insbesondere das Bügelelement der Zehenschiene und/oder der Mittelfußschiene, hingegen in einer Spannposition angeordnet sein, in der die Fußorthese, insbesondere das Bügelelement der Zehenschiene und/oder der Mittelfußschiene, elastisch verformt ist. Das Bügelelement der Zehenschiene und/oder der Mittelfußschiene, insbesondere eine Endabschnitt des Bügelelements, kann in der Spannposition gegenüber der Ruheposition in einer der ersten Korrekturkraft entgegengesetzten Richtung elastisch ausgelenkt sein. Beispielsweise kann das Bügelelement der Zehenschiene und/oder die Mittelfußschiene, insbesondere dessen Endabschnitt, in der Spannposition gegenüber der Ruheposition um wenigstens 0,2 cm, beispielsweise um wenigstens 0,3 cm oder wenigstens 0,5 cm oder wenigstens 1,0 cm, ausgelenkt oder translatorisch verschoben sein, insbesondere entlang der zu der ersten Korrekturkraft entgegengesetzten Richtung.

In der Spannposition kann das Bügelelement der Zehenschiene und/oder der Mittelfußschiene durch dessen elastische Verformung gespannt oder vorgespannt sein in Richtung der ersten Korrekturkraft. Die auf das Bügelelement der Zehenschiene oder der Mittelfußschiene wirkende Spannkraft oder Biegekraft kann der ersten Korrekturkraft und/oder Haltekraft entsprechen.

Um das Bügelelement der Zehenschiene oder der Mittelfußschiene kraftübertragend mit der zu schienenden Zehe oder dem zu schienenden Mittelfuß zu koppeln, kann das Bügelelement derart ausgebildet sein, dass dieses die Zehe oder den Mittelfuß zumindest teilweise umgreift. Mit anderen Worten kann das Bügelelement der Zehenschiene und/oder der Mittelfußschiene derart ausgestaltet sein, dass es in dem am Fuß befestigten Zustand die Zehe oder den Mittelfuß zumindest teilweise umgreift. Unter einem Umgreifen der Zehe oder des Mittelfußes wird vorliegend verstanden, dass das Bügelelement sich in dem am Fuß befestigten Zustand in Zehenumfangsrichtung entlang der Zehe erstreckt oder in Mittelfußumfangsrichtung entlang des Mittelfußes erstreckt. Vorzugsweise erstreckt sich das Bügelelement um die Zehe oder den Mittelfuß über ein Bogenmaß von wenigstens 1/2 π rad oder π rad um die Zehenlängsachse oder die Mittelfußlängsachse. Das bedeutet, dass sich das Bügelelement in Umfangsrichtung wenigstens entlang eines Viertels oder einer Hälfte des Umfangs einer Zehe oder des Mittelfußes erstreckt.

Im Hinblick auf die geometrische Ausgestaltung ist das Bügelelement vorzugsweise platten- und/oder schalenförmig ausgebildet. Beispielsweise kann das Bügelelement in seiner geometrischen Ausgestaltung riemen- oder bandförmig ausgebildet sein und insbesondere biegesteif sein. Entsprechend kann das Bügelelement mit einer für die Zehe vorgesehenen Kontaktfläche und einer gegenüberliegenden Auflagefläche ausgestaltet sein. In dem am Fuß befestigten Zustand der Fußorthese kann die Kontaktfläche des Bügelelements an der Zehe anliegen, insbesondere unmittelbar anliegen. Die Kontaktfläche kann hierbei in Form einer Wendefläche ausgestaltet sein, deren Orientierung, d.h. deren Flächennormale, sich entlang der Zehenlängsachse ändert und vorzugsweise auf die Zehenlängsachse zeigt. Auf diese Weise kann sich die Kontaktfläche und damit auch das Bügelelement entlang einer Schraubenlinie um die Zehenlängsachse erstrecken.

Gemäß einer Ausführungsform kann das Bügelelement derart ausgebildet sein, dass sich die Größe des Bügelelements, insbesondere dessen Spannweite entlang der Querachse der Fußorthese, an die Größe des zu behandelnden Fußes anpassen lässt. Hierzu kann das Bügelelement beispielsweise mehrteilig ausgeführt sein, wobei eine entsprechende Vielzahl von Teilen des Bügelelements miteinander gekoppelt sind und entlang der Querachse der Fußorthese relativ zueinander verschiebbar sind. Zur Größenanpassung des Bügelelements kann die Kopplung zwischen der Vielzahl von Teilen selektiv gesperrt oder freigegeben werden. In einem freigegebenen Zustand der Kopplung zwischen der Vielzahl von Teilen können diese relativ zueinander translatorisch verschoben werden, um eine gewünschte relative Position zueinander einzustellen. Daraufhin kann die Kopplung durch Kraft- und/oder Formschluss gesperrt werden, sodass eine relative translatorische Bewegung zwischen der Vielzahl von Teilen gesperrt ist.

Alternativ oder zusätzlich kann das Bügelelement der Zehenschiene und/oder der Mittelfußschiene entlang der Zehe oder entlang des Mittelfußes an einer Fußinnenseite verlaufen, insbesondere in Form eines Biegeschenkels, und mit Hilfe eines Stützbandes an der Zehe oder an dem Mittelfuß kraftübertragend befestigt sein. Hierzu kann das Stützband, das auch als Bandage bezeichnet werden kann, in Umfangsrichtung zumindest abschnittsweise an der Zehe oder dem Mittelfuß anliegen, wobei das Stützband mit dem Bügelelement verbunden ist, um so das Bügelelement form- und/oder kraftschlüssig relativ zu der Zehe oder dem Mittelfuß zu fixieren. Das Stützband kann in Form eines Riemens, einer Schlaufe, eines Bandes, eines Gurtes, etc. bereitgestellt sein.

Unter dem Begriff "Stützband" wird vorliegend und im Allgemeinen eine Komponente verstanden, die dazu eingerichtet und dafür vorgesehen ist, Belastungen aufzunehmen und zu übertragen, insbesondere Zugkräfte. Weiterhin kann es sich im Fall von Stützbändern vorliegend insbesondere um längenverstellbare Schlaufenelemente handeln. Entsprechend kann die Umfangslänge des Stützbandes durch einen Anwender einstellbar sein. Dadurch sind zum einen fußspezifische Anpassungen möglich und zum anderen kann ein effektiver Kraftfluss gewährleistet werden. Im Allgemeinen sind die Stützbänder entlang ihres Umfangs zugstarr, beziehungsweise im Wesentlichen zugstarr. Zusätzlich oder alternativ können die Stützbänder entlang ihres Umfangs elastisch oder abschnittsweise elastisch sein. Konkret können die Stützbänder in Form von zugstarren und/oder elastischen, beziehungsweise abschnittsweise elastischen Ringbandagen oder Schlaufenelementen vorliegen.

Das Stützband kann an einer lateralen Seite der Zehe oder des Mittelfußes anliegen und auf diese oder diesen die Korrekturkraft oder Haltekraft ausüben. Hierzu kann das Stützband an dessen Endabschnitten mittels des Bügelelements mit einer Zugkraft beaufschlagt sein. In dem befestigten Zustand der Fußorthese kann das Stützband zumindest teilweise um die Zehe oder den Mittelfuß in Umfangsrichtung angeordnet sein.

Gemäß einer Weiterbildung der Fußorthese kann das Drehgelenk dazu eingerichtet sein, in dem am Fuß befestigen Zustand Scherkräfte und/oder Biegekräfte parallel zu der ersten oder zweiten Korrekturkraft zwischen der Mittelfußschiene und der Zehenschiene zu übertragen. Somit kann das Drehgelenk über ein Zusammenwirken der ersten Korrekturkraft mit der Haltekraft die zweite Korrekturkraft generieren. Dadurch kann ferner der Betrag der zweiten Korrekturkraft durch einen Anwender variiert werden, indem die Zehenschiene oder die Mittelfußschiene, beispielsweise die Umfangslänge des oder der Stützbänder, durch den Anwender eingestellt beziehungsweise angepasst wird/werden.

Die Zehenschiene und/oder die Mittelfußschiene können/kann aus einem Kunststoff hergestellt sein, insbesondere aus einem Thermoplast, einem thermoplastischen Elastomer, etc. Die Zehenschiene und/oder die Mittelfußschiene können/kann durch ein additives Fertigungsverfahren oder ein Spritzgussverfahren hergestellt sein. Auch können die einzelnen Komponenten unterschiedliche Materialien, insbesondere Kunststoffe umfassen, die vorzugsweise unterschiedliche Werkstoffeigenschaften aufweisen. Die Verwendung eines additiven Fertigungsverfahrens oder eines Spritzgussverfahrens ermöglicht hierbei, dass die einzelnen Segmente integral ausgebildet sein können und trotzdem aus unterschiedlichen Materialien bestehen können und unterschiedliche Materialeigenschaften aufweisen können. Mit anderen Worten können die aus unterschiedlichen Materialien bestehenden Teile der einzelnen Komponenten integral oder stoffschlüssig miteinander verbunden sein.

In einer Weiterentwicklung kann die Fußorthese, insbesondere die Zehenschiene und/oder die Mittelfußschiene und/oder das Drehgelenk, innenseitig eine Auflage oder Beschichtung, insbesondere eine Polsterbeschichtung, beispielsweise eine polsternde Polyvinylchlorid(PVC)-Beschichtung oder eine Polyurethan(PU)-Beschichtung, aufweisen, wobei die Beschichtung insbesondere weicher ist, d.h. eine geringere Härte aufweist, als das die Beschichtung tragende Teil der Fußorthese. Die Beschichtung kann auf wenigstens einer der Komponenten der Fußorthese innenseitig aufgebracht sein. Der Begriff "innenseitig" betrifft jene Bereiche der Fußorthese, die im befestigten Zustand dem Fuß zugewandt sind. Die Polsterbeschichtung kann in einem additiven Verfahren als Feststoff oder Gel auf die Innenseiten der Fußorthese beziehungsweise der Schienen aufgebracht werden. Beispielsweise kann die Polsterbeschichtung durch Laminieren oder Kaschieren aufgebracht werden. Weiterhin kann ein geeignetes PVC-Material oder PU-Material aufgeschmolzen und auf die Schiene aufgepresst werden, um die Beschichtung zu erzeugen. Zusätzlich oder alternativ kann ein Auftrag der Beschichtung im flüssigen Zustand erfolgen, wobei der Auftrag mittels Tauchen, Rakeln, Walzenauftrag, Spritzverfahren, Schäumen oder einem anderen geeigneten Verfahren vorgenommen werden kann.

### Kurze Beschreibung der Figuren

Bevorzugte weitere Ausführungsformen der Erfindung werden durch die nachfolgende Beschreibung der Figuren näher erläutert. Dabei zeigen schematisch:
- Figur 1: eine perspektivische Ansicht auf eine Fußorthese in einem an einem Fuß eines Patienten befestigten Zustand;
- Figur 2 und 3: die in Figur 1 gezeigte Fußorthese in unterschiedlichen perspektivischen Ansichten, bei denen aus Übersichtsgründen der Fuß nicht dargestellt ist;
- Figur 4 bis 8: unterschiedliche Ansichten auf die in Figuren 1 bis 3 gezeigte Fußorthese in einem vom Fuß entkoppelten Zustand;
- Figur 9: einen Längsschnitt entlang eines Drehgelenks der in Figuren 1 bis 8 gezeigten Fußorthese;
- Figur 10: eine weitere Ausführungsform der Fußorthese in einer perspektivischen Ansicht, in der die Fußorthese an dem Fuß des Patienten befestigt ist; und
- Figur 11 und 12: die in Figur 10 gezeigte Fußorthese in einem vom Fuß entkoppelten Zustand.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

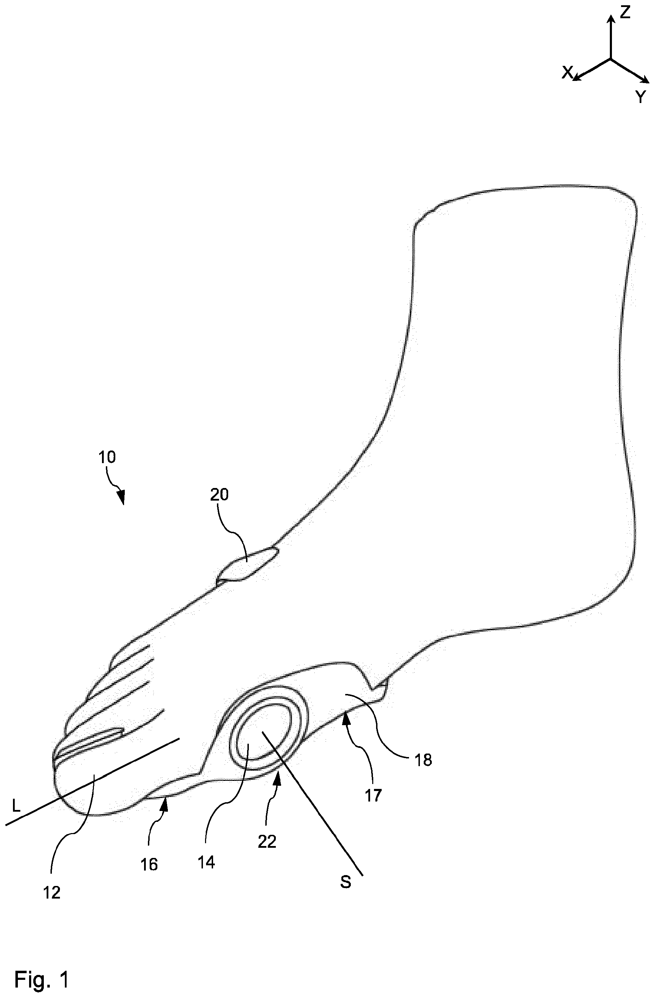

Figur 1 zeigt eine Ausführungsform einer Fußorthese 10 zur Korrektur von Fußfehlstellungen. Genauer ist die in Figur 1 gezeigte Fußorthese dafür vorgesehen, die als Hallux valgus bekannte pathologische Fehlstellung einer Großzehe 12, auch als Valgusstellung der Großzehe bezeichnet, und eines Großzehengrundgelenks 14, auch als Varusstellung des Großzehengrundgelenks bezeichnet, zu behandeln. Auch kann die hier gezeigte Fußorthese 10 zur Vorbeugung von Hallux valgus verwendet werden. Zur Vereinfachung wird nachstehend der Begriff "Großzehe" durch den Begriff "Zehe" abgekürzt und der Begriff "Großzehengrundgelenk" durch "Zehengrundgelenk".

Wie in Figur 1 gezeigt, ist die Fußorthese 10 dafür vorgesehen, an einem zu behandelnden Fuß in der Art einer Klammer, insbesondere Spannklammer, befestigt und in einer vorgegebenen Position fixiert zu werden. Die Fußorthese 10 ist derart ausgebildet und dazu eingerichtet, in dem am Fuß befestigten Zustand therapeutisch auf den Fuß einzuwirken, indem gezielt Korrekturkräfte in den Fuß, insbesondere im Bereich der Zehe 12 und des Zehengrundgelenks 14, eingeleitet werden, wie nachstehend genauer beschrieben wird.

Die gezeigte Fußorthese 10 ist zur Anwendung an dem rechten Fuß des Patienten vorgesehen. Zur Behandlung des linken Fußes des Patienten kann eine zu der in Figur 1 gezeigten Ausgestaltung spiegelsymmetrische Fußorthese eingesetzt werden.

Die Fußorthese 10 umfasst eine an der Zehe 12 befestigbare Zehenschiene 16 und eine im Bereich eines Mittelfußes befestigbare Mittelfußschiene 17, umfassend ein Ballensegment 18 und ein Mittelfußsegment 20. Die Zehenschiene 16 und die Mittelfußschiene 17 sind mittels eines Drehgelenks 22 relativ zueinander verschwenkbar gekoppelt. In der hier gezeigten Ausführungsform ist die Mittelfußschiene 17 durch das im Bereich des Zehengrundgelenks 14 anzuordnende Ballensegment 18 und das am oder im Bereich des Mittelfußes befestigbare Mittelfußsegment 20 gebildet.

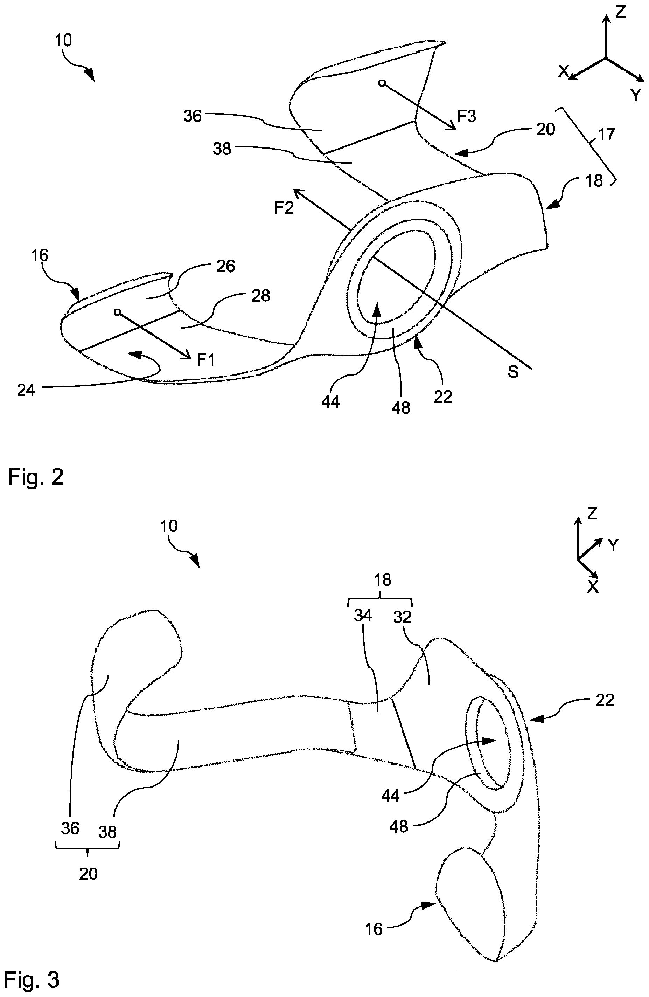

In dem am Fuß befestigten Zustand, wie in Figur 2 angedeutet, ist die Fußorthese 10 dazu eingerichtet, über die Zehenschiene16 eine erste Korrekturkraft F1 auf die Zehe 12 auszuüben, über das Drehgelenk 22 eine zu der ersten Korrekturkraft F1 entgegengesetzte und dazu beabstandete parallele zweite Korrekturkraft F2 auf das Zehengrundgelenk 14 auszuüben und über die Mittelfußschiene 17 eine Haltekraft F3, die eine dritte Korrekturkraft bilden kann, auf den Mittelfußbereich auszuüben, wobei die Haltekraft F3 in Richtung der ersten Korrekturkraft F1 zeigt und zu dieser beabstandet parallel angeordnet ist.

Die auf den Fuß einwirkenden Kräfte F1, F2, F3 bei Gebrauch der Fußorthese 10 sind in Figuren 2 dargestellt, in der aus Übersichtsgründen der behandelte Fuß nicht gezeigt ist. Zur Vereinfachung werden die entsprechenden Kräfte F1, F2, F3 als einzelne Vektoren dargestellt, wobei im Gebrauch diese über eine Kontaktfläche zwischen Fuß und Fußorthese 10 verteilt wirken.

Wie nachstehend im Detail beschrieben wird, sind die einzelnen Komponenten der Fußorthese 10, insbesondere die Zehenschiene 16 und die Mittelfußschiene 17 elastisch verformbar, wobei die unterschiedlichen auf den Fuß durch die Fußorthese 10 wirkenden Kräfte F1, F2, F3 in Form von durch elastische Verformung der Fußorthese 10 induzierte Spann- und/oder Biegekräfte bereitgestellt werden. Diese Ausgestaltung kann bewirken, dass auch beim Bewegen des Fußes und damit einhergehend bei Veränderung der Form des Fußes im Gebrauch die durch die Fußorthese 10 ausgeübten Kräfte F1, F2, F3 anhaltend auf den Fuß wirken. Auch kann so erreicht werden, dass bei einer Auslenkung der Zehe 12 relativ zu dem Mittelfuß, die auf diese wirkende Korrekturkraft angepasst wird, beispielsweise verstärkt wird, indem die elastische Verformung der Fußorthese, insbesondere der Zehenschiene 16 und/oder der Mittelfußschiene 17, zunimmt. Dies kann die therapeutische Wirkung der Fußorthese verbessern.

Die erste Korrekturkraft F1, die zweite Korrekturkraft F2 und die Haltekraft F3 sind relativ zueinander parallel oder im Wesentlichen parallel und beabstandet zueinander angeordnet. Hierbei zeigen die erste Korrekturkraft F1 und die Haltekraft F3 in mediale Richtung und sind parallel oder im Wesentlichen parallel zu einer Querachse Y der Fußorthese 10. Die zweite Korrekturkraft F2 zeigt in laterale Richtung. Die erste Korrekturkraft F1, die zweite Korrekturkraft F2 und die Haltekraft F3 sind weiterhin orthogonal oder im Wesentlichen orthogonal zu einer Längsachse X und einer Höhenachse Z der Fußorthese 10 angeordnet.

Die Zehenschiene 16 ist in Form eines Bügelelements ausgebildet, insbesondere eines Spann- oder Biegebügels, der in dem am Fuß befestigten Zustand die Zehe 12 umgreift. In der hier gezeigten Ausführungsform erstreckt sich die Zehenschiene 16 von einer medialen Seite des Fußes ausgehend von dem Ballensegment 18 über eine Zehenunterseite bis hin zu einer lateralen Seite der Zehe 12. Somit erstreckt sich die Zehenschiene 16 abschnittsweise entlang der Zehenunterseite. Alternativ kann sich die Zehenschiene 16 auch entlang einer Zehenoberseite erstrecken. In der hier gezeigten Ausführungsform erstreckt sich die Zehenschiene 16 über ein Bogenmaß von im Wesentlichen π rad um eine Zehenlängsachse L, sodass sich die Zehenschiene 16 von einer Seite der Zehe 12 und des Zehengrundgelenks 14 zu der entgegengesetzten Seite der Zehe 12 erstreckt, wie in Figur 1 gezeigt.

Die Zehenschiene 16 ist mit einer für die Zehe 12 vorgesehenen Kontaktfläche 24 ausgestaltet. Die Kontaktfläche 24 ist in Form einer Wendefläche ausgestaltet, deren Orientierung, d.h. deren Flächennormale, sich entlang der Zehenlängsachse L ändert und vorzugsweise auf die Zehenlängsachse zeigt. Auf diese Weise erstreckt sich die Kontaktfläche 24 und damit auch Die Zehenschiene 16 entlang einer Schraubenlinie um die Zehe 12.

Die Zehenschiene 16 ist dazu eingerichtet, in dem am Fuß befestigten Zustand Scherkräfte und/oder Biegekräfte in Richtung der ersten Korrekturkraft F1 zwischen dem Drehgelenk 22 und der zu behandelnden Zehe 12 zu übertragen, um so zur Generierung der ersten Korrekturkraft F1 beizutragen.

Um die erste Korrekturkraft F1 auf die Zehe 12 auszuüben, umfasst Die Zehenschiene 16 einen Zehenstützabschnitt 26, der in dem befestigten Zustand der Fußorthese 10 an einer lateralen Seite der Zehe 12, d.h. an einer in lateraler Richtung zeigenden Seite der Zehe 12, anliegt. Der Zehenstützabschnitt 26 ist durch einen distalen Endabschnitt der Zehenschiene 16 gebildet. Die Zehenschiene 16 umfasst ferner einen Zehenbasisabschnitt 28, der mit dem Zehenstützabschnitt 26 integral und stoffschlüssig verbunden ist und an diesen angrenzt, wie durch eine gepunktete Linie in Figur 2 angedeutet.

Die Zehenschiene 16, insbesondere der Zehenstützabschnitt 26, ist in der Art einer Biegefeder bereitgestellt. So wird die erste Korrekturkraft F1 in Form einer durch eine elastische Verformung der Zehenschiene 16 induzierte Spannkraft oder Biegekraft bereitgestellt. Mit anderen Worten ist die Zehenschiene 16 derart ausgestaltet, dass in dem am Fuß befestigten Zustand die Zehenschiene 16 in einer Spannposition angeordnet ist, in der die Zehenschiene 16 gegenüber einer Ruheposition des Zehensegments 16, in der die Zehenschiene 16 in einem vom Fuß entkoppelten Zustand der Fußorthese 10 angeordnet ist, elastisch ausgelenkt und zwar in einer der ersten Korrekturkraft F1 entgegengesetzten Richtung.

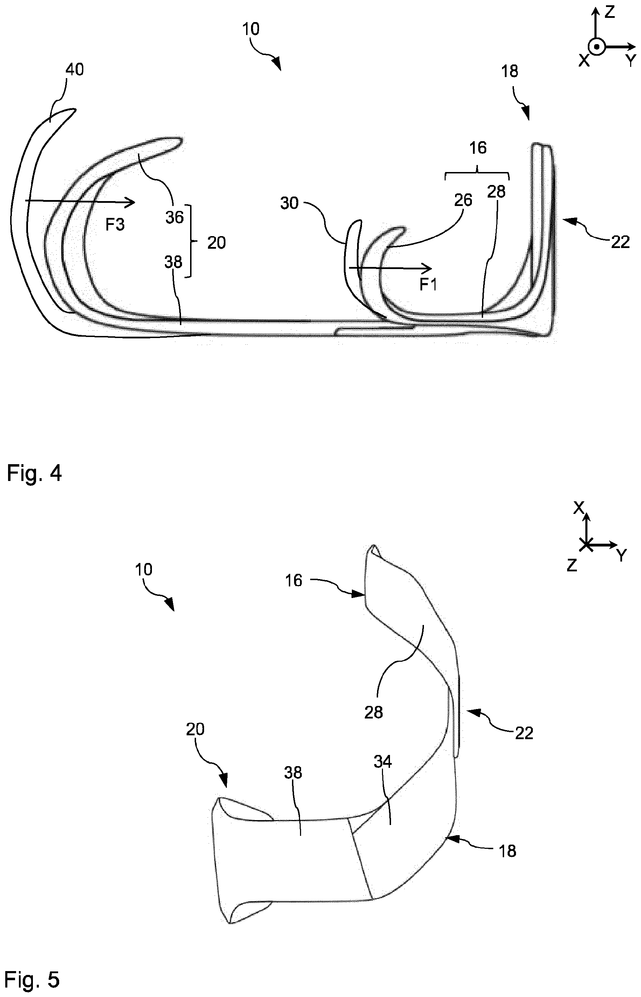

Zur Veranschaulichung dieser Eigenschaft der Zehenschiene 16 ist in Figur 4 die Fußorthese 10 in einem vom Fuß entkoppelten und daher freiliegenden Zustand gezeigt, in dem die Zehenschiene 16 in ihrer Ruheposition angeordnet ist. Weiterhin ist mithilfe einer gestrichelten Linie 30 ein Zustand der Zehenschiene 16 angedeutet, in dem diese in der Spannposition angeordnet ist, d.h. in dem am Fuß befestigten Zustand. Ein Endabschnitt des Zehenstützabschnitts 26 in der Spannposition ist gegenüber der Ruheposition um wenigstens 0,3 cm oder 0,5 cm, beispielsweise um wenigstens 1,0 cm, ausgelenkt und translatorisch verschoben entlang der zu der ersten Korrekturkraft F1 entgegengesetzten Richtung.

Das Ballensegment 18 ist in dem am Fuß befestigten Zustand der Fußorthese im Bereich des Zehengrundgelenks 14 angeordnet und liegt im Bereich des Zehengrundgelenks 14 an dem Fuß an. Das Ballensegment 18 umfasst einen Ballenstützabschnitt 32, der in dem befestigten Zustand der Fußorthese 10 im Bereich des Grundgelenkfußballens anliegt, im Speziellen medial und plantar an dem Fuß anliegt. Das Ballensegment 18 umfasst ferner einen Ballenbasisabschnitt 34, der denjenigen Teil des Ballensegments 18 bildet, der sich entlang der Fußsohle des Fußes erstreckt. Der Ballenbasisabschnitt 34 ist mit dem Ballenstützabschnitt 32 integral und stoffschlüssig verbunden und grenzt an diesen an, wie durch eine gepunktete Linie in Figur 3 angedeutet.

Das Ballensegment 18, insbesondere der Ballenstützabschnitt 32, ist in der Art einer Biegefeder bereitgestellt. So wird die zweite Korrekturkraft F2 in Form einer durch eine elastische Verformung des Ballensegments 18 und der Zehenschiene 16 induzierte Spannkraft oder Biegekraft bereitgestellt. Mit anderen Worten ist das Ballensegment 18 derart ausgestaltet, dass in dem am Fuß befestigten Zustand das Ballensegment 18 in einer Spannposition angeordnet ist, in der das Ballensegment gegenüber einer Ruheposition, in der das Ballensegment 18 in einem vom Fuß entkoppelten Zustand der Fußorthese 10 angeordnet ist, elastisch ausgelenkt ist in einer der zweiten Korrekturkraft F2 entgegengesetzten Richtung. Ein Endabschnitt des Ballenstützabschnitts 32 in der Spannposition kann gegenüber der Ruheposition um wenigstens 0,3 cm oder 0,5 cm ausgelenkt und translatorisch verschoben sein entlang der zu der zweiten Korrekturkraft F2 entgegengesetzten Richtung.

Das Ballensegment 18 ist mit dem Mittelfußsegment 20 kraft- und momentübertragend verbunden. Das Mittelfußsegment 20 ist am Mittelfuß des zu behandelnden Fußes befestigbar und teilweise gegenüberliegend zu dem Ballensegment 18, insbesondere dem Ballenstützabschnitt 32, angeordnet. Das Mittelfußsegment 20 ist in Form eines Bügels, insbesondere Spannbügels, bereitgestellt, der in dem am Fuß befestigten Zustand der Fußorthese 10 einen seitlichen Mittelfußbereich, insbesondere einen lateralen Mittfußbereich, teilweise umgreift. Das als Bügel ausgebildete Mittelfußsegment 20 kann dazu eingerichtet sein, in dem am Fuß befestigten Zustand Scherkräfte und/oder Biegekräfte, insbesondere in Richtung der Haltekraft, aufzunehmen und zwischen dem Ballensegment 18 und dem zu behandelnden Mittelfuß zu übertragen.

Mit anderen Worten ist die Mittelfußschiene 17 in Form eines Bügelelements ausgebildet, insbesondere in Form eines Spann- oder Biegebügels, der in dem am Fuß befestigten Zustand den Mittelfuß teilweise umgreift.

Um die Haltekraft in den Mittelfuß einzuleiten, umfasst das Mittelfußsegment 20 einen Mittelfußstützabschnitt 36, der in dem befestigten Zustand der Fußorthese 10 an dem Mittelfuß lateral anliegt. Der Mittelfußstützabschnitt 36 kann durch einen Endabschnitt des Mittelfußsegments 20 gebildet sein. Das Mittelfußsegment 20 umfasst ferner einen Mittelfußbasisabschnitt 38. Der Mittelfußbasisabschnitt 38 ist mit dem Mittelfußstützabschnitt 36 integral und stoffschlüssig verbunden und grenzt an diesen an, wie durch eine gepunktete Linie in Figur 2 angedeutet.

Das Mittelfußsegment 20, insbesondere der Mittelfußstützabschnitt 36 und der Mittelfußbasisabschnitt 38, ist in der Art einer Biegefeder bereitgestellt. So wird die Haltekraft F3 in Form einer durch eine elastische Verformung des Mittelfußsegments 20 induzierte Spannkraft oder Biegekraft bereitgestellt. Mit anderen Worten ist das Mittelfußsegment 20 derart ausgestaltet, dass in dem am Fuß befestigten Zustand das Mittelfußsegment 20 in einer Spannposition angeordnet ist, in der das Mittelfußsegment 20 gegenüber einer Ruheposition des Mittelfußsegments 20, in der das Mittelfußsegment 20 in einem vom Fuß entkoppelten Zustand der Fußorthese 10 angeordnet ist, elastisch ausgelenkt ist in einer der Haltekraft F3 entgegengesetzten Richtung. Diese Eigenschaft ist in Figur 4 veranschaulicht mithilfe einer weiteren gestrichelten Linie 40, die ein Zustand des Mittelfußsegments 20 andeutet, in dem dieses in der Spannposition angeordnet ist, d.h. in dem am Fuß befestigten Zustand. Ein Endabschnitt des Mittelfußstützabschnitts 36 in der Spannposition ist gegenüber der Ruheposition um wenigstens 0,5 cm oder 1,0 cm, beispielsweise um 2 cm, ausgelenkt und translatorisch verschoben entlang der zu der Haltekraft F3 entgegengesetzten Richtung.

Zum Fixieren der Fußorthese an dem Fuß kann die Mittelfußschiene 17 und/der die Zehenschiene 16 optional mit Bandagen oder Stützbändern versehen sein. Beispielsweise kann die Zehenschiene 16 mittels einer Bandage oder eines Stützbandes die in der Zehenschiene 16 eingespannte Zehe 12 form- und/oder kraftschlüssig relativ zu der Zehenschiene 16 fixieren. Hierzu kann die Bandage oder das Stützband in Umfangsrichtung zumindest abschnittsweise an der Zehe 12 anliegen und mit der Zehenschiene 16 verbunden sein. Weiterhin kann das Mittelfußsegment 20 mit einer zweiten Bandage oder einem zweiten Stützband versehen sein, die um den Mittelfuß in Umfangsrichtung geführt ist und an dessen Enden mit dem Mittelfußsegment 20 verbunden ist, um den Mittelfuß in dem Mittelfußsegment 20 form- und/oder kraftschlüssig zu fixieren.

Die Zehenschiene 16 und/oder die Mittelfußschiene 17, insbesondere das Ballensegment 18 und/oder das Mittelfußsegment 22, sind dünnwandig ausgestaltet. Insbesondere kann/können Die Zehenschiene 16 und/oder das Ballensegment 18 und/oder das Mittelfußsegment 22 aus plattenförmigen und/oder schalenförmigen Elementen aufgebaut sein oder bestehen, die eine maximale Wanddicke von weniger als 3 mm oder 2 mm oder 1 mm aufweisen.

Die Zehenschiene 16, das Ballensegment 18 und das Mittelfußsegment 22 sind vorzugsweise aus Kunststoff hergestellt. Insbesondere kommen hierfür Kunststoffe in Frage, die unter hoher Krafteinwirkung, d.h. die höher als die Korrekturkräfte F1, F2 und die Haltekraft F3 sind, oder unter Wärmeeinwirkung plastisch verformbar sind. Auf diese Weise kann die Fußorthese aufwandsreduziert an einen zu behandelnden Fuß in ihrer geometrischen Ausgestaltung angepasst werden. Auch lassen sich so die durch elastische Verformung bewirkten Korrektur- und Haltekräfte F1, F2, F3 anpassen.

Gemäß einer Weiterentwicklung kann wenigstens einer der Stützabschnitte 26, 32, 36 eine geringere Steifigkeit aufweisen, insbesondere gegenüber Scher- und/oder Biegekräfte in Richtung der ersten oder zweiten Korrekturkraft F1, F2, als der daran angrenzende Basisabschnitt 28, 34, 38. Hierzu kann der wenigstens eine Stützabschnitt 26, 32, 36 aus einem Material hergestellt sein, das gegenüber dem Material des angrenzenden Basiselements 28, 34, 38 ein geringeres Elastizitätsmodul oder eine geringere Härte, beispielsweise eine geringer Shore-Härte, aufweist.

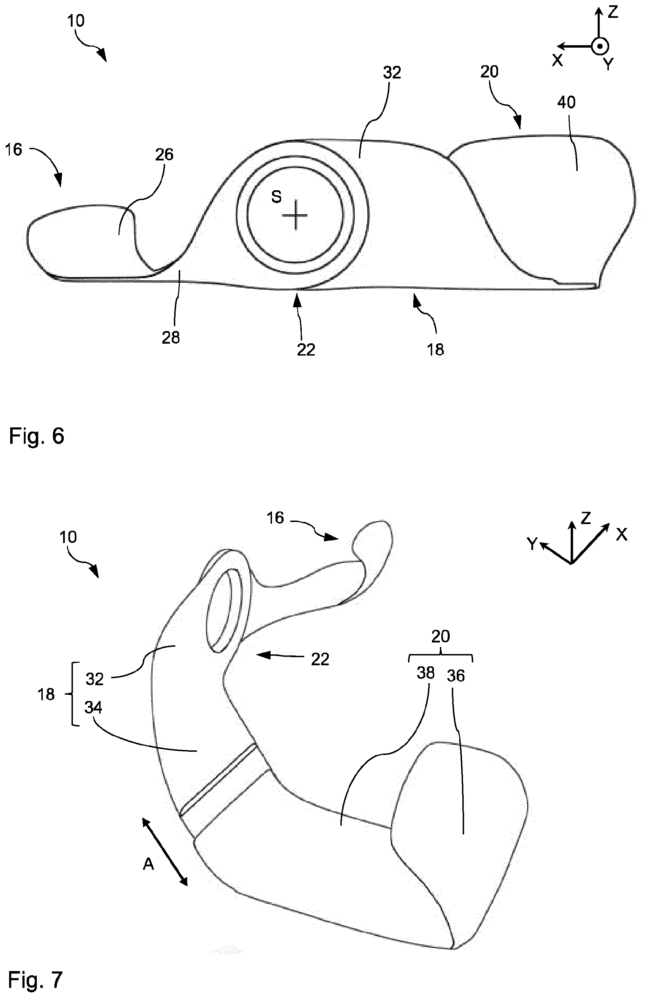

Wie in Figur 7 gezeigt, sind der Ballenbasisabschnitt 34 und der Mittelfußbasisabschnitt 38 derart gekoppelt, dass diese relativ zueinander translatorisch verschiebbar sind entlang einer Achse, die in einer Ebene mit der Längsachse X und einer Querachse Y der Fußorthese 10 liegt, aber quer zu diesen verläuft, wie in Figur durch den Pfeil A angedeutet. Mit anderen Worten sind die beiden Abschnitte 34, 38 relativ zueinander translatorisch verschiebbar entlang der Längsachse X und der Querachse Y der Fußorthese 10. Die Fußorthese 10 ist hierbei derart ausgestaltet, dass die beiden Abschnitte 34, 38 in einer gewünschten Position relativ zueinander kraft- und/oder formschlüssig fixierbar sind.

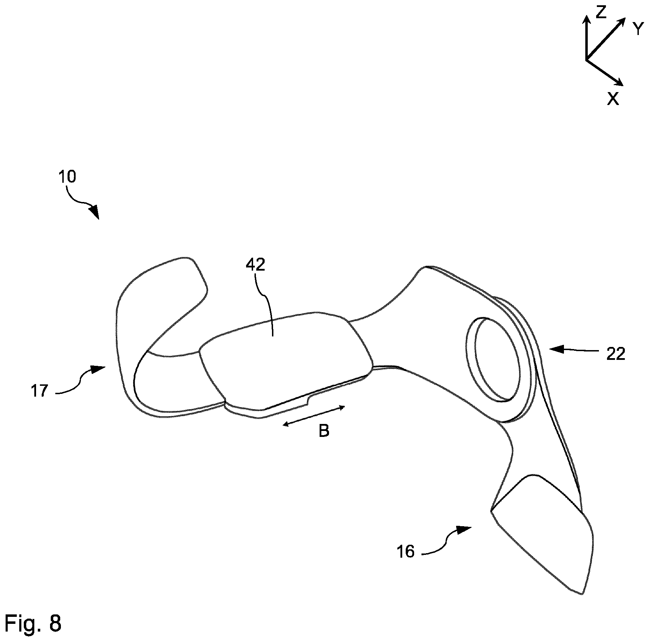

Optional kann die Fußorthese 10 ferner ein Fußkissen 42 umfassen, das lösbar mit dem Ballenbasisabschnitt 34 und/oder dem Mittelfußbasisabschnitt 38 verbunden werden kann und relativ zu diesen verschiebbar ist, um das Fußkissen 42 patientenspezifisch anzuordnen, wie in Figur 8 durch den Pfeil B angedeutet.

Wie vorangehend beschrieben, sind die Zehenschiene 16 und die Mittelfußschiene 17 mittels des Drehgelenks 22 relativ zueinander um die Schwenkachse S verschwenkbar gekoppelt. Das Drehgelenk 22 ist dazu eingerichtet, in dem an dem Fuß befestigten Zustand der Fußorthese 10 zu der ersten oder zweiten Korrekturkraft F1, F2 parallele Biegekräfte zwischen der Mittelfußschiene 17 und der Zehenschiene 16 zu übertragen, wobei die erste Korrekturkraft F1, die zweite Korrekturkraft F2 und die Haltekraft F3 in Form einer durch eine elastische Verformung der Zehenschiene 16 und der Mittelfußschiene 17 induzierte Biegekraft bereitgestellt sind.

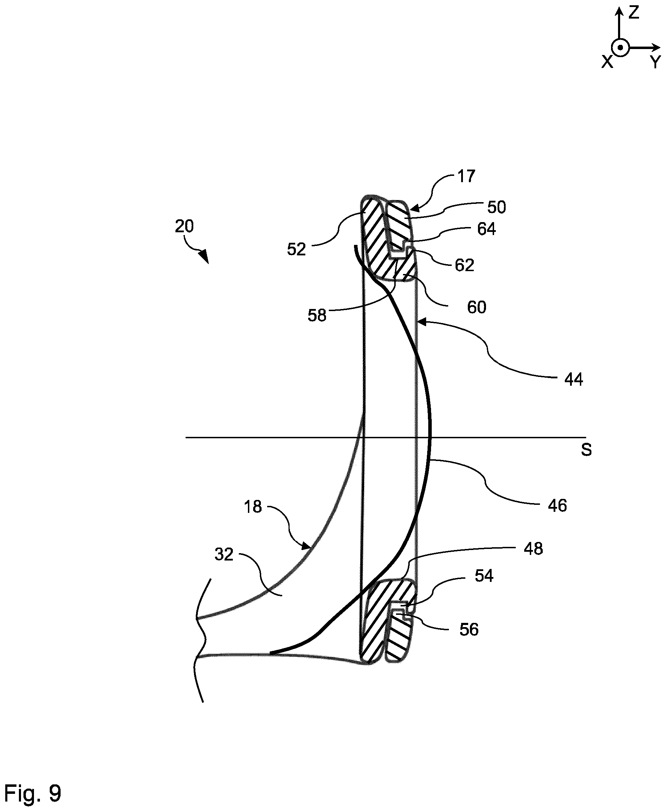

Zur Interaktion mit dem Zehengrundgelenk 14 des zu behandelnden Fußes, das heißt zum Ausüben der zweiten Korrekturkraft F2, ist die Fußorthese 10 mit dem Drehgelenk 22 ausgestattet. Die Ausgestaltung des Drehgelenks 22 wird nachstehend unter Bezugnahme auf Figur 9 spezifiziert, die einen Längsschnitt entlang der Schwenkachse S durch das Drehgelenk 22 zeigt.

Wie in Figur 9 veranschaulicht, ist das Drehgelenk 22 mit einer Ausnehmung 44 entlang seiner Schwenkachse S versehen und derart ausgebildet, dass in dem an dem Fuß befestigten Zustand der Fußorthese 10 ein seitlich ausbuchtender Teil 46 des Zehengrundgelenks 14 in der Ausnehmung 44 zumindest teilweise aufgenommen ist. Dies ist in Figur 9 durch die gestrichelte Linie mit dem Bezugszeichen "46" veranschaulicht. Der seitlich ausbuchtende Teil 46 des Zehengrundgelenks 14 kann eine Pseudoexostose sein oder bilden.

Das Drehgelenk 22 ist ein Hohlzapfendrehgelenk. Mit anderen Worten sind die das Drehgelenk 22 bildenden Komponenten entlang der Schwenkachse S hohl ausgebildet, sodass das Drehgelenk 22 um und entlang dessen Schwenkachse S mit der Ausnehmung 44 versehen ist. Das Drehgelenk ist derart ausgebildet, dass in dem befestigten Zustand der Fußorthese 10 das Drehgelenk 22 derart an dem Fuß und dem Zehengrundgelenk 14 angeordnet ist, dass das Drehgelenk 22 umlaufend um den seitlich ausbuchtenden Teil 46 des Zehengrundgelenks 12, beispielsweise um die Pseudoexostose, angeordnet ist, wobei der seitlich ausbuchtende Teil 46 des Zehengrundgelenks 14 zumindest teilweise in der Ausnehmung des Drehgelenks angeordnet ist.

Die Ausnehmung 44 ist in Form einer Durchgangsöffnung entlang der Schwenkachse S ausgebildet. Das Drehgelenk 22 ist derart ausgestaltet, dass in dem befestigten Zustand der seitlich ausbuchtende Teil 46 des Zehengrundgelenks sich entlang wenigstens 50 % oder wenigstens 70% oder wenigstens 80% der maximalen Breite des Drehgelenks erstreckt. In der hier gezeigten Ausführungsform ragt der seitlich ausbuchtende Teil 46 des Zehengrundgelenks 14 entlang der Schwenkachse S durch die Ausnehmung 44 hindurch. Das Drehgelenk hat eine maximale Breite entlang der Schwenkachse S, d.h. entlang der Querachse Y, von höchstens 1,0 cm oder 0,6 cm.

Das Drehgelenk 22 umfasst eine die Ausnehmung 44 begrenzende und radial um die Schwenkachse S angeordnete Seitenwand 48. Die Seitenwand 48 hat einen minimalen Krümmungsradius von 1 mm oder 2 mm oder 5 mm.

Die Ausnehmung 44 hat einen minimalen Durchmesser entlang einer Richtung quer zur Schwenkachse S von wenigstens 1,5 cm oder wenigstens 2,0 cm oder wenigstens 2,5 cm. Genauer beträgt der Durchmesser in der hier gezeigten Ausführungsform im Wesentlichen 3,0 cm.

In der gezeigten Konfiguration ist die Schwenkachse S des Drehgelenks 22 fluchtend oder im Wesentlichen fluchtend zu der Flexion-Extension-Gelenkachse des Zehengrundgelenks 14 angeordnet. Unter der Flexion-Extension-Gelenkachse wird diejenige Gelenkachse verstanden, um die die Zehe 12 bei Flexions- und Extensionsbewegungen verschwenkt wird relativ zum Mittelfuß. Hierzu ist das Drehgelenk 22 in dem am Fuß befestigten Zustand an der medialen Seite des Fußes angeordnet. Im Speziellen ist das Drehgelenk 22 derart ausgebildet, dass eine relative Schwenkbewegung zwischen der Zehenschiene 16 und der Mittelfußschiene 17 nur um die Schwenkachse S zugelassen ist.

Wie in Figur 9 gezeigt, ist das Drehgelenk 22 aus wenigstens zwei Komponenten gebildet, ist hierauf aber nicht beschränkt und kann in alternativen Ausführungsformen aus mehr als zwei Komponenten gebildet sein.

Genauer umfasst die Drehgelenk 22 ein mit der Zehenschiene16 gekoppeltes, insbesondere integral oder stoffschlüssig damit verbundenes, erstes Gelenkelement 50 und ein dazu komplementär ausgebildetes und im Eingriff stehendes zweites Gelenkelement 52, das mit der Mittelfußschiene 17, insbesondere dem Ballensegment 18, gekoppelt ist, insbesondere integral oder stoffschlüssig damit verbunden ist. Genauer ist das erste Gelenkelement 50 durch einen Endabschnitt des Zehenbasisabschnitts 28 gebildet und das zweite Gelenkelement 52 durch einen Endabschnitt des Ballenbasisabschnitts 38. In dem an dem Fuß befestigten Zustand ist das zweite Gelenkelement 52 entlang der Schwenkachse S zwischen dem Fuß und dem ersten Gelenkelement 50 angeordnet.

In der gezeigten Ausführungsform ist das erste Gelenkelement 50 in Form eines Gelenkrings bereitgestellt. Das zweite Gelenkelement 52 ist in Form eines hohlen Gelenkzapfens bereitgestellt, auf dem der Gelenkring, d.h. das erste Gelenkelement 50, entlang einer Führungsfläche geführt ist. Das erste und zweite Gelenkelement 50, 52 sind derart ausgebildet und stehen derart im Eingriff, dass diese entlang der Schwenkachse S und quer zu dieser formschlüssig gekoppelt sind.

Hierzu ist das zweite Gelenkelement 52 ist mit einer um die Schwenkachse S umlaufenden radialen Aufnahmenut 54 versehen, die mit einem dazu komplementär gestalteten Verbindungsring 56 des ersten Gelenkelements 50 im Eingriff steht. Die Aufnahmenut 54 erstreckt sich in radialer Richtung zu der Schwenkachse S derart, dass die Aufnahmenut 54 in beiden Richtungen entlang der Schwenkachse S seitlich begrenzt ist und in der radial nach außen zeigenden Richtung eine Öffnung aufweist, über die der Verbindungsring 56 in die Aufnahmenut 54 ragt. Mit anderen Worten hat die Aufnahmenut 54 im Längsschnitt entlang der Schwenkachse S im Profil eine im Wesentlichen U-förmige Kontakt- und Gleitfläche 58, auch als Führungsfläche bezeichnet, für den Verbindungsring 56. So sind die Aufnahmenut 54 und der Verbindungsring 56 in beiden Richtungen entlang der Schwenkachse S formschlüssig im Eingriff. Mit anderen Worten wird der Verbindungsring 56 in der Aufnahmenut 54 geführt. Auf diese Weise kann einem unbeabsichtigten Lösen der Verbindung zwischen dem ersten und dem zweiten Gelenkelement 50, 52 effektiv entgegengewirkt werden und gleichzeitig ein einfacher und robuster Aufbau der Gelenkeinheit sichergestellt werden. Alternativ kann das erste Gelenkelement mit einer Aufnahmenut und das zweite Gelenkelement mit einem dazu korrespondierenden Verbindungsring ausgebildet sein.

Das Drehgelenk 22 ist derart ausgestaltet, dass in dem am Fuß befestigten Zustand der Fußorthese 10, das zweite Gelenkelement 52 zwischen dem Fuß und dem ersten Gelenkelement 50 angeordnet ist, wobei ein Verbindungssteg 60 des zweiten Gelenkelements 52, der die Aufnahmenut 54 ausbildet, die Ausnehmung 44 umlaufend in radialer Richtung begrenzt. Der Verbindungssteg 60 erstreckt sich entlang der Schwenkachse S derart, dass der Verbindungssteg 60 in Axialrichtung des Drehgelenks 22, d.h. entlang der Schwenkachse S, überlappend zu dem ersten Gelenkelement 50 angeordnet ist und den Verbindungsring 56 umgreift. Ein Endabschnitt 62, der die Ausnehmung 44 seitlich begrenzt, ist hierbei in einer weiteren seitlich außen liegenden Ausnehmung 64 in dem ersten Gelenkelement 50 angeordnet, sodass ein bündiger seitlicher Abschluss des Drehgelenks 22 erreicht wird.

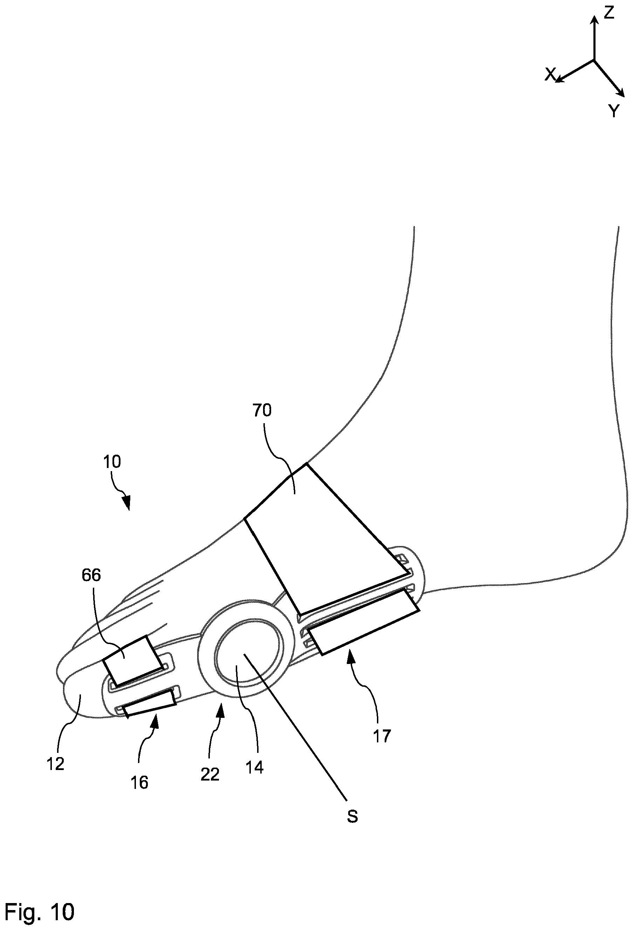

Figuren 10 bis 12 zeigen eine weitere Ausführungsform der Fußorthese, die sich gegenüber der in Figur 1 bis 9 gezeigten Ausführungsform durch die Ausgestaltung der Zehenschiene 16 und der Mittelfußschiene 17 unterscheidet. Die Ausgestaltung des Drehgelenks 22, insbesondere des ersten und des zweiten Gelenkelements 50, 52, ist entsprechend zu der in Figuren 9 gezeigten und in diesem Zusammenhang beschriebenen Ausgestaltung des Drehgelenks 22 in der voranstehend beschriebenen Ausführungsform.

Figur 10 zeigt die Fußorthese in einem am Fuß befestigten Zustand. Die Zehenschiene 16 umfasst einen an der Zeheninnenseite verlaufenden Zehenschenkel in der Form eines Bügelelements und ein daran gekoppeltes erstes Stützband 66 in Form einer Bandage. Das erste Stützband 66 ist um die Zehe 12 entlang dessen Umfangsrichtung geführt und an dessen Enden mit dem Zehenschenkel verbunden, um den Zehenschenkel relativ zu der Zehe 12 form- und/oder kraftschlüssig zu fixieren. Der Zehenschenkel bildet eine Biegefeder, die mittels des ersten Stützbandes 66 an der Zehe 12 befestigbar ist. Der Zehenschenkel umfasst Kopplungselemente 68, über die Endabschnitte des ersten Stützbandes 66 mit dem Zehenschenkel kraftschlüssig verbunden sind. Die Kopplungselemente 68 sind in der hier gezeigten Ausführungsform als Schlitzöffnungen bereitgestellt, durch welche das erste Stützband 66 beim Umschlingen der Zehe 12 geführt ist, um so die Fußorthese 10 am zu behandelnden Fuß zu fixieren und entsprechend die erste Korrekturkraft über das erste Stützband 66 in den Fuß einzuleiten.

Die Mittelfußschiene 17 umfasst einen an der Fußinnenseite verlaufenden Mittelfußschenkel in der Form eines Bügelelements und ein daran gekoppeltes zweites Stützband 70 in Form einer Bandage. Das Zweite Stützband 70 ist um den Mittelfuß entlang dessen Umfangsrichtung geführt und an dessen Enden mit dem Mittelfußschenkel verbunden, um den Mittelfußschenkel relativ zu dem Mittelfuß form- und/oder kraftschlüssig zu fixieren. Der Mittelfußschenkel bildet eine Biegefeder, die mittels des zweiten Stützbandes 70 an dem Mittelfuß befestigbar ist. Der Mittelfußschenkel umfasst weitere Kopplungselemente 72, über die Endabschnitte des zweiten Stützbandes 70 mit dem Zehenschenkel kraftschlüssig verbunden sind. Die weiteren Kopplungselemente 72 sind in der hier gezeigten Ausführungsform als Schlitzöffnungen bereitgestellt, durch welche das zweite Stützband 70 beim Umschlingen des Mittelfußes geführt ist, um so die Fußorthese 10 am zu behandelnden Fuß zu fixieren und entsprechend die Haltekraft über das zweite Stützband 70 in den Fuß einzuleiten.

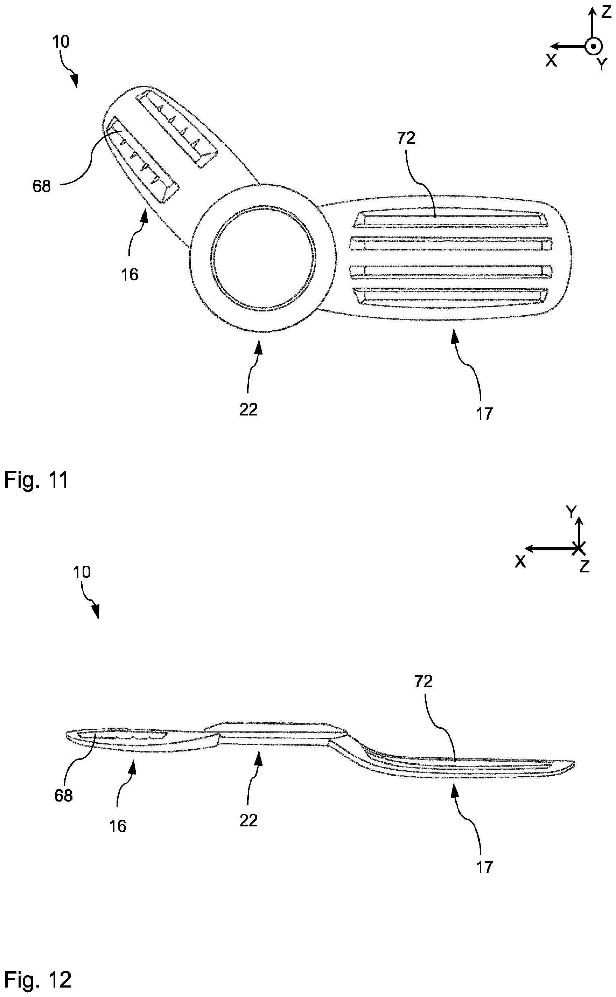

Figuren 11 und 12 zeigen die Fußorthese 10 in einem vom Fuß entkoppelten Zustand, in dem aus Übersichtsgründen die Stützbänder 66, 70 nicht gezeigt sind.

Das Drehgelenk 22 ist entsprechend dazu eingerichtet, in dem an dem Fuß befestigten Zustand der Fußorthese 10 zu der ersten und zweiten Korrekturkraft F1, F2 parallele Biegekräfte zwischen der Mittelfußschiene 17 und der Zehenschiene 16 zu übertragen, wobei die erste Korrekturkraft F1, die zweite Korrekturkraft F2 und die Haltekraft F3 in Form einer durch eine elastische Verformung der Zehenschiene 16, insbesondere des Zehenschenkels, und der Mittelfußschiene 17, insbesondere des Mittelfußschenkels, induzierte Biegekraft bereitgestellt sind.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

### Bezugszeichenliste

- 10: Fußorthese
- 12: Zehe
- 14: Zehengrundgelenk
- 16: Zehenschiene
- 17: Mittelfußschiene
- 18: Ballensegment
- 20: Mittelfußsegment
- 22: Drehgelenk
- 24: Kontaktfläche
- 26: Zehenstützabschnitt
- 28: Zehenbasisabschnitt
- 30: Spannposition der Zehenschiene
- 32: Ballenstützabschnitt
- 34: Ballenbasisabschnitt
- 36: Mittelfußstützabschnitt
- 38: Mittelfußbasisabschnitt
- 40: Spannposition des Mittelfußsegments
- 42: Fußkissen
- 44: Ausnehmung
- 46: seitlich ausbuchtende Teil des Zehengrundgelenks
- 48: Seitenwand
- 50: erstes Gelenkelement
- 52: zweites Gelenkelement
- 54: Aufnahmenut
- 56: Verbindungsring
- 58: Kontakt- und Gleitfläche
- 60: Verbindungssteg
- 62: Endabschnitt
- 64: weitere Ausnehmung
- 66: erstes Stützband
- 68: Kopplungselemente
- 70: zweites Stützband
- 72: weitere Kopplungselemente

- F1: erste Korrekturkraft
- F2: zweite Korrekturkraft
- F3: Haltekraft
- S: Gelenkachse

## Patentansprüche

1. Fußorthese (10) zur Korrektur von Fußfehlstellungen, insbesondere zur Behandlung von Hallux valgus, umfassend eine an einer Zehe (12) befestigbare Zehenschiene (16) und eine im Bereich eines Mittelfußes befestigbare Mittelfußschiene (17), die mittels eines Drehgelenks (22) relativ zueinander verschwenkbar gekoppelt sind, wobei die Fußorthese (10) dazu eingerichtet ist, in einem an einem Fuß ordnungsgemäß befestigten Zustand über die Zehenschiene (16) eine erste Korrekturkraft (F1) auf die Zehe (12) auszuüben und über das Drehgelenk (22) eine zu der ersten Korrekturkraft (F1) entgegengesetzte zweite Korrekturkraft (F2) auf ein Zehengrundgelenk (14) auszuüben, wobei das Drehgelenk (22) mit einer Ausnehmung in der Form einer Durchgangsöffnung (44) entlang einer Schwenkachse (S) des Drehgelenks (22) versehenes ist und derart ausgebildet ist, dass in dem an dem Fuß befestigten Zustand der Fußorthese (10) ein seitlich ausbuchtender Teil (46) des Zehengrundgelenks (14) in der Durchgangsöffnung (44) aufgenommen ist, wobei das Drehgelenk ein mit der Zehenschiene (16) verbundenes erstes Gelenkelement (50) in der Form eines Gelenkrings und ein mit der Mittelfußschiene (17) verbundenes zweites Gelenkelement (52) in der Form eines Gelenkzapfens umfasst, die entlang der Schwenkachse (S) und quer zu der Schwenkachse (S) des Drehgelenks (20) formschlüssig im Eingriff stehen, wobei der Gelenkzapfen (52) mit einer Aufnahmenut (54) versehen ist, in der ein dazu korrespondierender Verbindungsring (56) des Gelenkrings (50) geführt ist, und wobei der Gelenkzapfen (52) an einem proximalen Ende einen umlaufenden radialen Absatz (62) umfasst, der in Richtung der Schwenkachse (S) eine formschlüssige Verbindung zwischen dem Gelenkring (50) und dem Gelenkzapfen (52) bereitstellt und in einer korrespondierend ausgebildeten weiteren Ausnehmung (64) am Gelenkring (50) aufgenommen ist.

2. Fußorthese nach Anspruch 1, wobei das Drehgelenk (22) ein Hohlzapfendrehgelenk ist.

3. Fußorthese nach einem der Ansprüche 1 oder 2, wobei das Drehgelenk (22) eine maximale Breite entlang der Schwenkachse (S) von höchstens 0,6 cm aufweist.

4. Fußorthese nach einem der Ansprüche 1 bis 3, wobei in dem an dem Fuß befestigten Zustand der Fußorthese (10) der seitlich ausbuchtende Teil (46) des Zehengrundgelenks (14) sich entlang wenigstens 50 % der maximalen Breite des Drehgelenks (22) entlang der Schwenkachse (S) erstreckt.

5. Fußorthese nach einem der Ansprüche 1 bis 4, wobei in dem an dem Fuß befestigten Zustand der Fußorthese (10) der seitlich ausbuchtende Teil (46) des Zehengrundgelenks (14) durch die Durchgangsöffnung entlang der Schwenkachse (S) hindurchragt.

6. Fußorthese nach einem der Ansprüche 1 bis 5, wobei das Drehgelenk (22) derart ausgestaltet ist, dass eine die Ausnehmung (44) begrenzende Seitenwand (48) des Drehgelenks (22) einen minimalen Krümmungsradius von 1 mm oder 2 mm oder 5 mm aufweist.

7. Fußorthese nach einem der Ansprüche 1 bis 6, wobei ein minimaler Durchmesser der Ausnehmung (44) um die Schwenkachse (S) wenigstens 2,0 cm oder wenigstens 2,5 cm beträgt.

8. Fußorthese nach einem der Ansprüche 1 bis 7, wobei in dem an dem Fuß befestigten Zustand der Fußorthese (10) die Schwenkachse (S) des Drehgelenks (22) mit einer Grundgelenkachse des Zehengrundgelenks (14) im Wesentlichen fluchtet.

9. Fußorthese nach einem der Ansprüche 1 bis 8, wobei das Drehgelenk (22) derart ausgebildet ist, dass eine relative Schwenkbewegung zwischen der Zehenschiene (16) und der Mittelfußschiene (17) nur um die Schwenkachse (S) zugelassen ist.

10. Fußorthese nach einem der Ansprüche 1 bis 9, wobei das erste Gelenkelement (50) mit der Zehenschiene (16) integral oder stoffschlüssig verbunden ist, und das zweite Gelenkelement (52) mit der Mittelfußschiene (17) integral oder stoffschlüssig verbunden ist.

11. Fußorthese nach einem der Ansprüche 1 bis 10, wobei in dem an dem Fuß befestigten Zustand das mit der Mittelfußschiene (17) verbundene zweite Gelenkelement (52) entlang der Schwenkachse (S) zwischen dem Fuß und dem mit der Zehenschiene (16) verbundenen ersten Gelenkelement (50) angeordnet ist.

12. Fußorthese nach einem der Ansprüche 1 bis 11, die dazu eingerichtet ist, in dem an dem Fuß befestigten Zustand über die Mittelfußschiene (17) eine zu der zweiten Korrekturkraft (F2) entgegengesetzte Haltekraft (F3) auf den Mittelfuß auzuüben, wobei
das Drehgelenk (22) dazu eingerichtet ist, in dem an dem Fuß befestigten Zustand der Fußorthese (10) zu der ersten oder zweiten Korrekturkraft (F1, F2) parallele Biegekräfte zwischen der Mittelfußschiene (17) und der Zehenschiene (16) zu übertragen, und wobei
in dem an dem Fuß befestigten Zustand die erste Korrekturkraft (F1), die zweite Korrekturkraft (F2) und die Haltekraft (F3) in Form einer durch eine elastische Verformung der Zehenschiene (16) und der Mittelfußschiene (17) induzierte Biegekraft bereitgestellt sind.

## Claims

1. Foot orthosis (10) for correcting malpositions of the foot, in particular for the treatment of hallux valgus, comprising a toe splint (16) that can be fastened to a toe (12) and a metatarsal splint (17) that can fastened in the mid-foot region, which are coupled pivotably relative to each other by a pivot joint (22), wherein, when the foot orthosis (10) is correctly position on the foot, it is designed to apply a first correcting force (F1) to the toe (12) via the toe splint (16) and to apply a second correcting force (F2) opposite to the first corrective force (F1) to a metatarsophalangeal joint (14) via the pivot joint (22), wherein the pivot joint (22) is provided with a recess in the form of a through-opening (44) along a pivot axis (S) of the pivot joint (22) and is formed such that, when the foot orthosis (10) is fastened to the foot, a laterally bulging portion (46) of the metatarsophalangeal joint (14) is accommodated in the through-opening (44), wherein the pivot joint comprises a first joint element (50) in the form of a joint ring connected to the toe splint (16) and a second joint element (52) in the form of a pivot pin connected to the metatarsal splint (17), which are interlockingly engaged along the pivot axis (S) and transversely to the pivot axis (S) of the pivot joint (20), wherein the pivot pin (52) is provided with a locating groove (54), in which a corresponding connecting ring (56) of the joint ring (50) is guided, and wherein the pivot pin (52) comprises a circumferentially radial recess (62) at a proximal end, which provides an interlocking connection between the joint ring (50) and the pivot pin (52) in the direction of the pivot axis (S) and is accommodated in a correspondingly formed further recess (64) on the joint ring (50).

2. Foot orthosis according to claim 1, wherein the pivot joint (22) is a hollow pin pivot joint.

3. Foot orthosis according to any one of claims 1 or 2, wherein the pivot joint (22) has a maximum width along the pivot axis (S) of at most 0.6 cm.

4. Foot orthosis according to any one of claims 1 to 3, wherein, when the foot orthosis (10) is fastened to the foot, the laterally bulging portion (46) of the metatarsophalangeal joint (14) extends along the pivot axis (S) along at least 50 % of the maximum width of the pivot joint (22).

5. Foot orthosis according to any one of claims 1 to 4, wherein, when the foot orthosis (10) is fastened to the foot, the laterally bulging portion (46) of the metatarsophalangeal joint (14) protrudes through the through-opening along the pivot axis (5).

6. Foot orthosis according to any one of claims 1 to 5, wherein the pivot joint (22) is configured such that a side wall (48) of the pivot joint (22) delimiting the recess (44) has a minimum radius of curvature of 1 mm or 2 mm or 5 mm.

7. Foot orthosis according to any one of claims 1 to 6, wherein a minimum diameter of the recess (44) about the pivot axis (S) is at least 2.0 cm or at least 2.5 cm.

8. Foot orthosis according to any one of claims 1 to 7, wherein, when the foot orthosis (10) is fastened to the foot, the pivot axis (S) of the pivot joint (22) is substantially aligned with a metatarsophalangeal joint axis of the metatarsophalangeal joint (14).

9. Foot orthosis according to any one of claims 1 to 8, wherein the pivot joint (22) is formed such that a relative pivoting movement between the toe splint (16) and the metatarsal splint (17) is only permitted about the pivot axis (5).

10. Foot orthosis according to any one of claims 1 to 9, wherein the first joint element (50) is integrally or materially connected to the toe splint (16), and the second joint element (52) is integrally or materially connected to the metatarsal splint (17).

11. Foot orthosis according to any one of claims 1 to 10, wherein, in the foot-fastened state, the second joint element (52) that is connected to the metatarsal splint (17) is arranged along the pivot axis (S) between the foot and the first joint element (50) connected to the toe splint (16).

12. Foot orthosis according to any one of claims 1 to 11, which is designed to apply a holding force (F3) opposite to the second corrective force (F2) to the mid-foot via the metatarsal splint (17) in the foot-fastened state, wherein
the pivot joint (22) is designed, when the foot orthosis (10) is fastened to the foot, to transmit bending forces between the metatarsal splint (17) and the toe splint (16) parallel to the first or second corrective force (F1, F2), and wherein,
in the foot-fastened state, the first corrective force (F1), the second corrective force (F2) and the holding force (F3) are provided in the form of a bending force induced by an elastic deformation of the toe splint (16) and the metatarsal splint (17).

## Revendications

1. Orthèse de pied (10) destinée à la correction de déformations du pied, en particulier destinée au traitement de l'hallux valgus, comprenant une attelle pour orteils (16) pouvant être fixée à un orteil (12) et une attelle métatarsienne (17) pouvant être fixée dans la zone d'un métatarse, qui sont couplées de manière pivotante l'une par rapport à l'autre au moyen d'une articulation rotative (22), dans laquelle l'orthèse de pied (10) est conçue pour exercer une première force de correction (F1) sur les orteils (12) dans un état fixé correctement à un pied par le biais de l'attelle pour orteils (16) et exercer une seconde force de correction (F2) opposée à la première force de correction (F1) sur une articulation de base des orteils (14) par l'intermédiaire de l'articulation rotative (22), dans laquelle l''articulation rotative (22) est dotée d'un évidement sous la forme d'une ouverture traversante (44) le long d'un axe de pivotement (S) de l'articulation rotative (22) et est configurée de telle sorte qu'à l'état fixé au pied de l'orthèse de pied (10) une partie renflée latéralement (46) de l'articulation de base des orteils (14) est réceptionnée dans l'ouverture traversante (44), dans laquelle l'articulation rotative comprend un premier élément d'articulation (50) en forme d'anneau d'articulation relié à l'attelle pour orteils (16) et un second élément d'articulation (52) en forme de pivot d'articulation relié à l'attelle métatarsienne (17) qui se mettent en prise par complémentarité de formes le long de l'axe de pivotement (S) et transversalement à l'axe de pivotement (S) de l'articulation rotative (20), dans laquelle le pivot d'articulation (52) est doté d'une rainure de réception (54) dans laquelle un anneau de liaison (56) de l'anneau d'articulation (50) correspondant à celle-ci est guidé, et dans laquelle le pivot d'articulation (52) comprend un épaulement radial périphérique (62) au niveau d'une extrémité proximale qui, dans la direction de l'axe de pivotement (5), fournit une liaison par complémentarité de formes entre l'anneau d'articulation (50) et le pivot d'articulation (52) et est réceptionné au niveau de l'anneau d'articulation (50) dans un autre évidement (64) configuré de manière correspondante.

2. Orthèse de pied selon la revendication 1, dans laquelle l'articulation rotative (22) est une articulation rotative à pivot creux.

3. Orthèse de pied selon la revendication 1 ou 2, dans laquelle l'articulation rotative (22) présente une largeur maximale le long de l'axe de pivotement (S) d'au plus 0,6 cm.

4. Orthèse de pied selon l'une quelconque des revendications 1 à 3, dans laquelle à l'état fixé au pied de l'orthèse de pied (10) la partie renflée latéralement (46) de l'articulation de base des orteils (14) s'étend le long de l'axe de pivotement (S) le long d'au moins 50 % de la largeur maximale de l'articulation rotative (22).

5. Orthèse de pied selon l'une quelconque des revendications 1 à 4, dans laquelle à l'état fixé au pied de l'orthèse de pied (10) la partie renflée latéralement (46) de l'articulation de base des orteils (14) fait saillie le long de l'axe de pivotement (S) à travers l'ouverture traversante.

6. Orthèse de pied selon l'une quelconque des revendications 1 à 5, dans laquelle l'articulation rotative (22) est conçue de telle sorte qu'une paroi latérale (48) de l'articulation rotative (22) délimitant un évidement (44) présente un rayon de courbure minimal de 1 mm ou 2 mm ou 5 mm.

7. Orthèse de pied selon l'une quelconque des revendications 1 à 6, dans laquelle un diamètre minimal de l'évidement (44) autour de l'axe de pivotement (S) est d'au moins 2,0 cm ou d'au moins 2,5 cm.

8. Orthèse de pied selon l'une quelconque des revendications 1 à 7, dans laquelle à l'état fixé au pied de l'orthèse de pied (10) l'axe de pivotement (S) de l'articulation rotative (22) s'aligne sensiblement avec un axe d'articulation de base de l'articulation de base des orteils (14).

9. Orthèse de pied selon l'une quelconque des revendications 1 à 8, dans laquelle l'articulation rotative (22) est configurée de telle sorte qu'un mouvement de pivotement relatif entre l'attelle pour orteils (16) et l'attelle métatarsienne (17) n'est autorisé qu'autour de l'axe de pivotement (S).

10. Orthèse de pied selon l'une quelconque des revendications 1 à 9, dans laquelle le premier élément d'articulation (50) est relié intégralement ou par complémentarité de matières à l'attelle pour orteils (16) et le second élément d'articulation (52) est relié intégralement ou par complémentarité de matières à l'attelle métatarsienne (17).

11. Orthèse de pied selon l'une quelconque des revendications 1 à 10, dans laquelle à l'état fixé au pied le second élément d'articulation (52) relié à l'attelle métatarsienne (17) est disposé le long d'un axe de pivotement (S) entre le pied et le premier élément d'articulation (50) relié à l'attelle pour orteils (16).

12. Orthèse de pied selon l'une quelconque des revendications 1 à 11, qui est conçue pour exercer sur le métatarse à l'état fixé au pied par le biais de l'attelle métatarsienne (17) une force de retenue (F3) opposée à la seconde force de correction (F2), dans laquelle
l'articulation rotative (22) est conçue pour transférer à l'état fixé au pied de l'orthèse de pied (10) des forces de flexion parallèles à la première ou la seconde force de correction (F1, F2) entre l'attelle métatarsienne (17) et l'attelle pour orteils (16), et dans laquelle
à l'état fixé au pied la première force de correction (F1), la seconde force de correction (F2) et la force de retenue (F3) sont fournies sous la forme d'une force de flexion induite par le biais d'une déformation élastique de l'attelle pour orteils (16) et de l'attelle métatarsienne (17).
